(19) Europäisches Patentamt
European Patent Office
Office européen des brevets

(11) **EP 3 448 250 B1**

(12) **EUROPEAN PATENT SPECIFICATION**

(45) Date of publication and mention
of the grant of the patent:
**29.07.2020 Bulletin 2020/31**

(51) Int Cl.:
***A61B 5/04*** (2006.01)

(21) Application number: **17721430.1**

(86) International application number:
**PCT/GB2017/051152**

(22) Date of filing: **25.04.2017**

(87) International publication number:
**WO 2017/187160 (02.11.2017 Gazette 2017/44)**

(54) **MAGNETOMETER FOR MEDICAL USE**

MAGNETOMETER ZUR MEDIZINISCHE VERWENDUNG

MAGNETOMTÈRE POUR USAGE MÉDICAL

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **25.04.2016 GB 201607121**

(43) Date of publication of application:
**06.03.2019 Bulletin 2019/10**

(73) Proprietor: **Creavo Medical Technologies Limited Coventry CV4 8HS (GB)**

(72) Inventors:
• **VARCOE, Benjamin Thomas Hornsby**
  **Leeds LS2 9JT (GB)**
• **DIMAMBRO, David Diamante**
  **Leeds LS2 9DF (GB)**
• **WATSON, David Ian**
  **Leeds LS2 9DF (GB)**
• **GRANT, Richard Theodore**
  **Leeds LS2 9DF (GB)**

(74) Representative: **Dehns**
  **St. Bride's House**
  **10 Salisbury Square**
  **London EC4Y 8JD (GB)**

(56) References cited:
**WO-A2-2014/011940      US-A1- 2004 260 169**
**US-A1- 2013 079 622      US-A1- 2015 150 475**

• **SLAWOMIR TUMANSKI: "REVIEW ARTICLE; Induction coil sensors-a review", MEASUREMENT SCIENCE AND TECHNOLOGY, IOP, BRISTOL, GB, vol. 18, no. 3, 1 March 2007 (2007-03-01), pages R31-R46, XP020118574, ISSN: 0957-0233, DOI: 10.1088/0957-0233/18/3/R01**

## Description

The present invention relates to methods and apparatus for medical magnetometry, and in particular to methods and apparatus for processing a signal from a magnetometer for medical use, such as for use as a cardiac magnetometer.

It can be useful in many medical situations to be able to measure magnetic fields relating to or produced by the human body for diagnostic purposes. For example, the heart's magnetic field contains information that is not contained in an ECG (Electro-cardiogram), and so a magneto cardiogram scan can provide different and additional diagnostic information to a conventional ECG.

Modern cardiac magnetometers are built using ultra-sensitive SQUID (Superconducting Quantum Interference Device) sensors. However, SQUID magnetometers are very expensive to operate as they require cryogenic cooling. Their associated apparatus and vacuum chambers are also bulky pieces of equipment. This limits the suitability of SQUID magnetometers for use in a medical environment, for example because of cost and portability considerations.

Another known form of magnetometer is an induction coil magnetometer. Induction coil magnetometers have the advantage over SQUID magnetometers that cryogenic cooling is not required, they are relatively inexpensive and easy to manufacture, they can be put to a wide range of applications and they have no DC sensitivity.

Further magnetometers are known from US-2015/150475-A1, US-2004/260169-A1, and WO-2014/011940-A2.

However, induction coil magnetometers have not been widely adopted for magneto cardiography because magneto cardiography requires low field (<nT), low frequency (<100Hz) sensing, and common induction coil magnetometer designs that can achieve such sensitivities are too large to be practical for use as a cardiac probe.

The Applicants have addressed these problems in their earlier application WO2014/006387, which discloses a method and apparatus for detecting and analysing medically useful magnetic fields that uses an induction coil or coils of a specific configuration to detect the magnetic field of a subject.

Notwithstanding this, the Applicants believe that there remains scope for alternative arrangements and improvements to the design and use of magnetometers for medical use, and in particular for cardio magnetic imaging.

According to a first aspect of the present invention, there is provided a method of using a magnetometer system to analyse the magnetic field of a region of a subject's body as claimed in claim 1.

According to a second aspect of the present invention, there is provided a magnetometer system for medical use as claimed in claim 13.

The present invention is concerned with a method of analysing the magnetic field of a region of a subject, such as their heart. In the present invention, plural repeating periods of the time varying magnetic field of a region of a subject's body are detected, digitised and averaged overall plural periods. In contrast to existing arrangements, in the present invention, the system is configured such that the noise present in the signal to be digitised is greater than about 25% of the interval between digitisation levels of the digitiser. As will be discussed further below, the Applicants have found that, counter-intuitively, arranging for the noise to be relatively large in this manner is beneficial, and allows, for example, "wanted" signals (information in the signals) that would otherwise be smaller than the minimum signal detectable by the digitiser to be detected.

It should be noted that the present invention goes against the conventional approach of attempting to maximise the signal to noise ratio of the system, e.g. using passive shielding and/or active cancellation, but instead optimises the amount of noise present such that signal information (features) can be extracted using what would conventionally be considered to be an insufficiently sensitive detector system.

In particular, the Applicants have recognised that providing a relatively large amount of noise effectively increases the peak-to-peak amplitude of the signal. This in turn means that, where the periodic signal of interest is smaller than the interval between digitisation levels of the digitiser, the "noisy" signal will cause the digitiser to transition between adjacent levels more often, thereby increasing the information content of the digitised signal. The overall effect of this is that, by taking multiple readings of the periodic signal and averaging over multiple periods, a useful signal can be extracted even when the periodic signal of interest is smaller than the digitisation level interval of the digitiser.

It will be appreciated therefore that the present invention provides an improved magnetometer system for medical use.

The magnetometer system of the present invention can be used as a system and probe to detect any desired magnetic field produced by a subject (by the human (or animal) body). It is preferably used to detect (and analyse) the time varying magnetic field of (or produced by) a region of the subject's body, such as their bladder, heart, head or brain, womb or a foetus. Thus it may be, and is preferably, used to detect magnetic fields relating to the bladder, pregnancy, the brain, or the heart. In a preferred embodiment, the magnetometer is used for (and configured for) one or more of: magnetocardiography, magnetoencephalography, analysis and detection of bladder conditions (e.g. overactive bladder), analysis and detection of foetal abnormalities, and detection and analysis of pre-term labour.

In a particularly preferred embodiment the magnetometer is used as a cardiac magnetometer and to detect and analyse the magnetic field of a subject's heart.

Thus, in an embodiment there is provided a method of analysing the magnetic field of a subject's heart.

In an embodiment, there is provided a cardiac magnetometer system for analysing the magnetic field of a subject's heart.

The one or more detectors of the present invention may be configured to detect the time varying magnetic field of a region of a subject's body in any suitable and desired manner.

The magnetometer system of the present invention may comprise a single detector. In this case, the detector will be moved over the subject (e.g. the subject's chest) to take readings from different positions in use.

However, in one preferred embodiment, the magnetometer system comprises plural detectors.

Where the magnetometer system comprises plural detectors, some or all of the detectors may be arranged in a two dimensional array, e.g. and preferably at least 7, e.g. 7-50 (or more), preferably at least 16, e.g. 16-50 (or more) detectors arranged in a two dimensional array. In this case, the or each detector array is preferably configured such that when positioned appropriately over a subject (e.g. a subject's chest or other region of the subject's body) the detector array can take readings from a suitable set of sampling positions without the need to further move the array over the subject. Accordingly, the number (and configuration) of detectors in the or each array is preferably selected so as to provide an appropriate number of sampling points and/or an appropriate coverage for the region of the subject's body in question. For example, an increased number of detectors may be provided where it is desired to measure the time-varying magnetic field of a region of a subject's body other than the heart. The or each array can otherwise have any desired configuration, such as being a regular or irregular array, a rectangular or circular array (e.g. formed of concentric circles), etc.

The magnetometer system may comprise a single layer of detectors, or may comprise plural layers of one or more detectors, e.g. and preferably 2-10 (or more) layers, i.e. one above the other.

In one such embodiment, each detector layer comprises a single detector. In this case, then again, the magnetometer will be moved over the subject (e.g. the subject's chest) to take readings from different positions in use. However, in a preferred embodiment, one or more or all of the detector layers comprise plural detectors, e.g. arranged in a two dimensional array, with one or more or each array preferably arranged as discussed above for the two dimensional array arrangement.

In these embodiments, one or more or each detector in each detector layer may be aligned with one or more or each detector in one or more or all of the other layers or otherwise (e.g. anti-aligned), as desired.

Where the magnetometer system comprises plural detectors, some or all of the detectors may be connected, e.g. in parallel and/or in series. Connecting plural detectors in series will have the effect of increasing the induced voltage for a given magnetic field strength. Connecting plural detectors in parallel will have the effect of reducing the thermal noise (Johnson noise) in the detectors. Preferably, a combination of series and parallel connections is used to optimise the balance of voltage and noise performance of the detectors.

In an embodiment, one or more or each detector in the magnetometer system is arranged in a gradiometer configuration, i.e. where two detectors are co-axially aligned (in the direction orthogonal to the plane in which each coil's windings are arranged), and where the signal from each of the coils is summed, e.g. to provide a measure of a change in the magnetic field in space.

The or each detector in the magnetometer system may comprise any suitable detector for detecting a time varying magnetic field.

The or each detector is preferably configured to be sensitive to signals between 1 Hz and 60 Hz, as this is the frequency range of the relevant magnetic signals of the heart. The detectors are preferably optimised for sensitivity to signals at around 30 Hz, as 30 Hz is the principle frequency component of a human heart beat.

The or each detector is preferably sensitive to magnetic fields in the range 1-150 pT.

In a preferred embodiment, each detector in the magnetometer system comprises an induction coil. Thus, an induction coil or coils (i.e. a coil that is joined to an amplifier at both ends) is preferably used to detect the magnetic field of the subject (e.g. of the subject's heart). In this case, each coil may be configured as desired.

Each coil preferably has a maximum outer diameter less than 7 cm, preferably between 4 and 7 cm. By limiting the outer diameter of the coil to 7 cm or less, a coil having an overall size that can achieve a spatial resolution that is suitable for medical magnetometry (and in particular for magneto cardiography) is provided. In particular, this facilitates a medically applicable diagnostic using 16 to 50 sampling positions (detection channels) to generate an image. (As discussed above, and as will be appreciated by those skilled in the art, the data for each sampling position can, e.g., be collected either by using an array of coils, or by using one (or several) coils that are moved around the chest to collect the data.) In a preferred embodiment, coils of 7 cm diameter are used.

Each coil preferably has a non-magnetically active core (i.e. the coil windings are wound around a non-magnetically active core), such as being air-cored. This helps to reduce the noise in use. However, magnetically active, such as ferrite or other magnetic material, cores may be used if desired.

In one preferred embodiment, each coil corresponds to the arrangement described in the Applicants' earlier application WO2014/006387. Such coils can be used to provide a medical magnetometer that can be portable, relatively inexpensive, usable at room temperature and without the need for magnetic shielding, and yet can still provide sufficient sensitivity, accuracy and resolution to be medically useful.

Thus, each coil preferably has a configuration such that the ratio of the coil's length to its outer diameter is at least 0.5. Each coil preferably has a configuration such that the ratio of the coil's inner diameter to its outer diameter is 0.5 or less.

As described in the Applicants' earlier application WO2014/006387, the combination of these two requirements for the induction coil's configuration makes the coil relatively more sensitive to magnetic field components along the axis of the coil. This results in a higher output voltage from the coil for a given axial magnetic component, and helps the spatial resolution of the coil as the coil is relatively more sensitive to components extending vertically through the centre of the coil when it is placed over a subject's body (e.g. over a subject's chest), and thus can provide a directional pick up. The Applicants have recognised that it is the vertical components of the magnetic field generated by a region of a subject's body (e.g. by a subject's heart) that it is of particular interest to detect.

This coil configuration can provide an increase in the output voltage generated by the coil for the magnetic field components of interest without adversely affecting (and indeed even with reducing) the signal to noise ratio.

In a preferred embodiment, the ratio of the coil's inner diameter to its outer diameter, Di:D, is less than 0.5:1. In a particularly preferred embodiment, the ratio of the coil's inner diameter to its outer diameter, Di:D, is also greater than or equal to 0.3:1. Thus, the ratio of the coil's inner diameter to its outer diameter, Di:D, is preferably in the range 0.3:1 to 0.5:1, preferably 0.3:1 to <0.5:1. Most preferably it is substantially 0.425:1. These coil configurations have been found to give the lowest noise figure for the measurements of interest.

The ratio of the coil's length to its outer diameter, I:D, is preferably 0.5:1 or greater. It is preferably not more than unity (1:1), preferably in the range 0.5:1 to 0.8:1, and most preferably substantially 0.69:1. These configurations have been found to optimise the coil structure for measuring the axial component of the magnetic field (the component along the axis of the coil).

Thus, in a particularly preferred embodiment, the or each coil has the following configuration:

$4\ cm \le D \le 7\ cm$

$$\frac{l}{D} \approx 0.69$$

and

$$\frac{Di}{D} \approx 0.425$$

where:

D is the outer diameter of the coil
I is length of the coil and
Di is the inner diameter of the coil.

The outer diameter D affects the signal noise floor. A larger outer diameter D gives a lower noise floor. An outer diameter D in the range 4 cm ≤ D ≤ 7 cm (and with its other parameters as set out above) gives a noise floor between 0.8 pT to 0.2 pT.

The number of turns on the coil will be determined by the wire radius and the coil length I. The wire radius can be selected as desired to determine the voltage output: a smaller wire radius will increase the voltage output but at the expense of increased coil resistance. A preferred wire radius is 0.2 mm to 1 mm, preferably 0.5 mm. Any suitable conductor can be used for the wire.

A preferred number of turns for the or each coil is 1000 to 8000, preferably 2000. The winding density (the ratio of the cross-sectional area of the winding to the cross-sectional area of the wire) is preferably in the range 0.5 to 1, most preferably 1.

However, the or each coil need not comprise the optimised coil in accordance with WO2014/006387, and may have any suitable and desired configuration.

In another preferred embodiment, each detector in the magnetometer system comprises a planar (flat) coil, that is, a coil with plural turns arranged in a (single) plane. Utilising planar coils is beneficial, particularly where as discussed above the magnetometer system comprises plural detectors arranged in plural layers, since this can result in a magnetometer system having a size, shape, and weight that is more suitable for medical magnetometry (and in particular for magneto cardiography).

Thus, in a particularly preferred embodiment, one or more, and preferably an array, of planar coils is used to detect the time varying magnetic field of a subject's heart.

In these embodiments, each planar coil may be configured as desired.

Each planar coil preferably has a maximum outer diameter less than 7 cm, preferably less than 6 cm, preferably between 2 and 6 cm. Again, this provides a detector that can achieve a spatial resolution that is suitable for medical magnetometry (and in particular for magneto cardiography).

Each planar coil is preferably configured such that all of its turns are arranged in a single (the same) plane (e.g. are coplanar) or on a single (the same) surface.

Each planar coil may comprise, for example, a conductor, e.g. a wire or conductor track, arranged in a (two-dimensional, preferably flat) spiral or the like (e.g. spirangle). Any suitable conductor may be used for the planar coil, such as copper, gold, silver, carbon nanotubes, graphene, or similar.

The Applicants have recognised that, in these embodiments, it can be beneficial to increase the width of the conductor (conductor track) (its thickness in the direction parallel to the plane in which the planar coil's plural turns are arranged), and correspondingly to reduce the spacing (the gap) between each of the turns of the planar coil (in the direction parallel to the plane in which the planar coil's plural turns are arranged). This has the effect of reducing the resistance of the coil, while increasing the "useful" signal collecting area of the coil.

Increasing the height of the conductor (conductor track) (its thickness in the direction orthogonal to the plane in which the coil's plural turns are arranged) also has the effect of reducing the resistance of the coil. However, increasing the height of the conductor (conductor track) can affect the minimum spacing between turns that it is feasible to achieve in practice, e.g. depending on the particular manufacturing process used (for example, where chemical etching techniques are used, increasing the conductor height can increase the minimum spacing (the minimum gap) between each of the turns of the planar coil that it is feasible to achieve in practice).

In a preferred embodiment, the conductor (conductor track) has a width (a thickness in the (radial) direction parallel to the plane in which the planar coil's plural turns are arranged and perpendicular to the longitudinal direction of the track) of around 1 mm or less, preferably between around 0.1 and 1 mm, more preferably between around 0.3 and 0.5 mm.

Equally, in a preferred embodiment, the turns of the planar coil are spaced apart radially (in the direction parallel to the plane in which the planar coil's plural turns are arranged) by around 1 mm or less, preferably around 0.1 mm or less, more preferably around 0.01 mm or less.

The conductor (conductor track) is preferably relatively flat in the direction orthogonal to the plane in which the coil's plural turns are arranged. In a preferred embodiment, the conductor has a thickness (in the direction orthogonal to the plane in which the coil's plural turns are arranged) of around 0.2 mm or less, preferably 0.1 mm or less, more preferably 0.05 mm or less. The conductor thickness is preferably not less than 0.035 mm.

The Applicants have found that these value ranges are achievable in practice and provide planar coils that are capable of producing a useful output signal.

The planar (spiral) coil may comprise any desired number of turns, such as 2 or more turns.

Each planar (spiral) coil may have an inner diameter in the range 4-35 mm, more preferably 15-22 mm.

Preferably, each planar coil comprises a (spiral) conductor arranged on or within an electrically insulating substrate. The insulating substrate preferably supports the conductor, and is accordingly preferably substantially rigid. Any suitable insulator can be used for the insulating substrate, such as glass, plastic, reinforced plastic, etc. The substrate may also comprise a printed circuit board (PCB) material, such as FR4 and the like.

Each conductor (conductor track) may be formed in any suitable manner using any suitable technique, such as chemical etching, laser etching, etc. Laser etching can be used to achieve particularly small gaps between the turns of the planar coil (e.g. gaps as small as 3-5 $\mu$m).

In a preferred embodiment, each planar coil further comprises a magnetic core. This has been found to improve performance of the planar coils.

A magnetic core may be located, for example, within (e.g. at the axial centre of) each (spiral) planar coil, or otherwise adjacent to each (spiral) planar coil. Each magnetic core may have a diameter in the range 4-35 mm, more preferably 15-22 mm.

In these embodiments, any suitable magnetically active core material may be used. The magnetic core is preferably made from a material with a high relative permeability such as a ferrite or other magnetic material.

In a preferred embodiment, the magnetic core is made from a magnetic amorphous metal alloy and/or a nano-crystalline material. These materials can exhibit very high magnetic permeabilities, but can be lighter than other magnetic materials such as iron powder. This can beneficially reduce the overall weight of the magnetometer system.

It is believed that the idea of using an array of planar coils to measure the time-varying magnetic field of a region of a subject's body is new and advantageous in its own right.

Thus, in an embodiment the magnetometer system comprises:
one or more arrays of planar coils for detecting the time varying magnetic field of a region of a subject's body, each coil having a maximum outer diameter less than 7 cm.

In an embodiment, the method comprises:
using one or more arrays of planar coils to detect the time varying magnetic field of a region of a subject's body, each coil having a maximum outer diameter less than 7 cm.

The or each array may include any number of planar coils, e.g. and preferably at least 7, e.g. 7-50 (or more), preferably at least 16, e.g. 16-50 (or more) planar coils arranged in a two dimensional array (e.g. and preferably as described above).

In a particularly preferred embodiment, the or each array comprises 30-45 planar coils, preferably 37 planar coils, arranged in a two dimensional array. This number of planar coils has been found to provide particularly good spatial coverage for the human heart.

The one or more arrays of planar coils may comprise a single layer (a single array) of planar coils, or may comprise plural layers (plural arrays) of planar coils, e.g. and preferably 2-10 (or more) layers (arrays), i.e. one above the other, e.g. as described above.

In a particularly preferred embodiment, the one or more arrays of planar coils comprises 20-120 layers (arrays) of planar coils, preferably 40-90 layers (arrays), i.e. one above the other. This has been found to result in a magnetometer system that produces a useful signal, while having a weight that is suitable for medical magnetometry (and in particular for magneto cardiography).

In these embodiments, each layer may comprise its own substrate (e.g. PCB layer), i.e. each layer may comprise plural planar coils arranged on or within a single (electrically insulating) substrate (e.g. PCB). Alternatively, plural layers of planar coils may be arranged on or within a single (electrically insulating) substrate (e.g. multi-layer PCB). This can beneficially reduce the overall weight of the magnetometer system.

One or more or each planar coil in each array may be aligned with one or more or each planar coil in one or more or all of the other arrays or otherwise (e.g. anti-aligned), as desired. Some or all of the planar coils may be connected, e.g. in parallel and/or in series and/or may be arranged in a gradiometer configuration, e.g. and preferably as described above. Connecting plural planar coils in series has the effect of increasing sensitivity. Connecting plural planar coils in parallel reduces the effects of Johnson noise in the system. A combination of series and parallel connections may be (and is preferably) used to select (control) the amount of noise in the system (e.g. as is described in more detail below).

Each planar coil in the or each array is preferably configured as described above.

The digitiser of the present invention may comprise any suitable digitiser that is operable to digitise (convert) an analogue signal received from the one or more detectors into a digital signal, e.g. for further processing and averaging, etc. The digitiser should (and preferably does) convert a voltage or current generated in the one or more detectors (coils) by the magnetic field into a digital signal.

In a preferred embodiment, the magnetometer system comprises a digitiser coupled to each detector (each coil) and configured to digitise a signal from the detector. Where the system includes plural detectors, each detector may have its own, respective and separate, digitiser (i.e. there will be as many digitisers as there are detectors), or some or all of the detectors may share a digitiser.

The or each digitiser may be directly connected to the or each respective detector, or more preferably, the or each digitiser may be connected to the or each respective detector via an amplifier. Thus in a preferred embodiment, the magnetometer system includes one or more detection amplifiers, preferably in the form of a microphone amplifier (a low impedance amplifier), connected to one or more or each detector, e.g. to the ends of each coil. The or each detection amplifier is preferably then connected to a digitiser or digitisers.

The or each amplifier may be configured to have any suitable and desired amplification level. The or each amplifier may, for example, amplify the signal (including the noise) received from the or each detector by around 1000 times (60 dB) or more.

In a preferred embodiment, the magnetometer system is arranged such that the detector (e.g. coil) and amplifier (that is coupled to the detector (coil)) are arranged together in a sensor head or probe which is then joined by a wire to the remaining components of the magnetometer system to allow the sensor head (probe) to be spaced from the remainder of the magnetometer system in use.

The or each digitiser should be (and preferably is) operable to convert (to digitise) a received analogue signal into one of plural digitisation levels (plural discrete values). The digitiser may have any suitable and desired resolution, such as 8-bit, 10-bit, 12-bit, 14-bit, 16-bit, 18-bit, 20-bit, 22-bit, 24-bit, etc., resolution, and correspondingly may be capable of converting the signal into any (plural) number of digitisation levels (plural discrete values), such as $2^8$, $2^{10}$, $2^{12}$, $2^{14}$, $2^{16}$, $2^{18}$, $2^{20}$, $2^{22}$, $2^{24}$, etc. digitisation levels.

The plural digitisation levels (plural discrete values) are preferably equally spaced apart, preferably over some maximum range. As will be appreciated, the interval (spacing) between each of the plural digitisation levels corresponds to the minimum measurable value of a variation in the analogue signal.

The or each digitiser should (and preferably does) have some frequency, i.e. rate at which it converts the analogue signal into one of the plural discrete values.

In a preferred embodiment, the or each digitiser comprises an analogue to digital converter (ADC).

The signal or signals may be averaged over plural periods as desired, and the averaging circuitry may comprise any suitable and desired circuitry for averaging the digitised signal or signals over plural periods.

In a preferred embodiment, the digitised signal or signals, i.e. received from the digitiser or digitisers, are averaged over plural periods, i.e. over plural cycles of the periodic signal.

In a preferred embodiment, a trigger is provided and used for gating the signal (i.e. for identifying and dividing the periodic signal into its plural repeating periods). The trigger should be, and preferably is, synchronised with the time varying magnetic field of the region of the subject's body. For example, where the magnetometer is used to analyse the magnetic field of a subject's heart, then the signal is preferably averaged over a number of heart beats, and an ECG or Pulse Ox trigger from the test subject may be used as a detection trigger for the signal acquisition process.

Thus, in a preferred embodiment, a trigger is used to identify each repeating period of the periodic signal, and then the signal is averaged over the plural identified periods.

Other arrangements would, of course, be possible.

The averaged signal may be used to analyse the time varying magnetic field in any manner as desired. Preferably, the averaged signal(s) are subjected to appropriate signal processing, for example to generate false colour images of the magnetic field.

Thus, in a preferred embodiment, the averaged signal or signals are used to provide an output indicative of the time varying magnetic field. This preferably comprises providing a display indicative of the time varying magnetic field, e.g. displaying an image indicative of the time varying magnetic field on a display. Most preferably, the averaged signal or signals are used to provide a false colour image or images indicative of the time varying magnetic field, and the false colour image or images are displayed on a display.

In the present invention, the signal or signals that is or are digitised by the or each digitiser includes a periodic signal produced by one or more of the one or more detectors due to the time varying magnetic field of the region of the subject's body and noise.

The periodic signal produced by the one or more of the one or more detectors due to the time varying magnetic field of the region of the subject's body will include one or plural (different) signal features, i.e. one or plural (different) attributes or parts of the signal (that may or may not be of interest). For example, in the case of a signal produced due to the time varying magnetic field of a subject's heart, the signal may include (signal features corresponding to) the P wave, the QRS wave and/or the T wave, but may include other signal features.

Where the signal includes plural (different) signal features, then one or more or all of the signal features may be signal features of interest, but also one or more of the signal features may not be (may be other than) signal features of interest. For example, in the case of a signal produced by the time varying magnetic field of a subject's heart, the P wave, the QRS wave and/or the T wave may be signal features of interest, and other signal features may not be of interest.

The periodic signal produced by the one or more of the one or more detectors due to the time varying magnetic field of the region of the subject's body (preferably (immediately) after amplification and/or (immediately) prior to digitisation) includes (useful/wanted) signal features that are smaller than the interval between digitisation levels of the digitiser, i.e. signal features that would otherwise be smaller than the minimum signal detectable by the digitiser (as discussed above, a particularly advantageous feature of the present invention is that such signal features are detected). Preferably, no part of the useful/wanted signal features (of interest) (preferably (immediately) after amplification and/or (immediately) prior to digitisation) are larger than the interval between digitisation levels of the digitiser. There may or may not be other (non-useful or uninteresting) signal features present in the periodic signal that may have any size (and that may, for example, be larger than the interval between digitisation levels of the digitiser).

Thus, for example, the (useful/wanted) signal features (preferably (immediately) after amplification and/or (immediately) prior to digitisation) are smaller than the interval between digitisation levels of the digitiser, and may be smaller than about 75% of the interval between digitisation levels of the digitiser, smaller than about 50% of the interval between digitisation levels of the digitiser, and/or smaller than about 25% of the interval between digitisation levels of the digitiser.

Accordingly, in a preferred embodiment, the signal level amplitude (of signal features of interest) is significantly lower than the quantisation level of the digitiser.

Similarly, the (useful/wanted) features in the periodic signal (preferably (immediately) after amplification and/or (immediately) prior to digitisation) are preferably smaller than the noise in each signal (and again, a particularly advantageous feature of the present invention is that such signal features can be (and preferably are) detected).

Accordingly, in a preferred embodiment the signal to noise ratio of the signal feature(s) of interest that is or are digitised by the or each digitiser (preferably (immediately) after amplification and/or (immediately) prior to digitisation) is less than one, i.e. less than 0 dB, less than -10 dB, less than -20 dB, less than -30 dB, less than -40 dB, less than -50 dB, less than -60 dB, less than -70 dB, less than -80 dB, less than -90 dB, and/or less than -100 dB, less than -110 dB, less than -120 dB, less than -130 dB, less than -140 dB, and/or less than -150 dB.

For example, the signal to noise ratio of the signal features of interest that is or are digitised by the or each digitiser (preferably (immediately) after amplification and/or (immediately) prior to digitisation) may be around -55dB excluding external environmental noise, or around -120 dB when external environmental noise for a "typical" noise environment is included. However, as will be appreciated by those having skill in the art, the noise environment, and therefore the signal to noise ratio, may be subject to significant variation.

In the present invention, the magnetometer system is arranged such that the noise in each signal that is provided (input) to the digitiser for digitising is greater than about 25% of the interval between digitisation levels of the digitiser. Preferably, the noise amplitude of the signal (or, where, e.g., the noise comprises white noise or other Gaussian noise, the standard deviation of the noise amplitude of the signal) provided to the digitiser is greater than about 25% of the interval between adjacent digitisation levels of the digitiser (of the (voltage) spacing between adjacent digitisation levels of the digitiser). As will be appreciated by those having skill in the art, the term "noise" as used herein is preferably intended to mean the amplitude of the noise voltage (e.g. at a defined termination impedance) or the equivalent noise power, and may be defined and/or measured, e.g. in terms of a peak to peak value, quasi-peak value, or standard deviation.

In a preferred embodiment, the noise in each signal immediately prior to the signal being digitised is greater than about 25% of the interval between adjacent digitisation levels of the digitiser. That is, the magnetometer system is preferably configured such that the noise in each signal that is (presented to and) digitised by the digitiser is greater than about 25% of the interval between adjacent digitisation levels of the digitiser.

Correspondingly, where the signal(s) from the detector(s) are amplified before digitisation, the noise in each signal should be, and is preferably, greater than about 25% of the interval between digitisation levels of the digitiser immediately after amplification. That is, the magnetometer system is preferably configured such that the noise in each signal that is produced by the amplifier is greater than about 25% of the interval between digitisation levels of the digitiser.

The noise that is controlled in the present invention to be greater than about 25% of the interval between digitisation levels of the digitiser comprises detector noise and other system noise such as electronics noise.

Thus, in one embodiment, the magnetometer system is arranged such that the total noise in each signal is greater than about 25% of the interval between digitisation levels of the digitiser.

The total noise may take any value that is greater than about 25% of the interval between the digitisation levels. However higher levels of noise may mean that more periods of the signal must be averaged in order to obtain an output signal with a useful information content. This in turn may mean that a longer signal acquisition period is required. However, excessively long acquisition periods may be undesirable in practical medical situations.

In a preferred embodiment, the (total) noise in each signal should be less than around 1m times the digitisation interval, i.e. less than 120 dB. This may result in a sufficiently short acquisition period to be medically useful. The (total) noise in each signal is preferably less than 100 dB, less than 80 dB, less than 60 dB, less than 40 dB and/or less than 20 dB.

In a preferred embodiment, the total noise present in the signal or signals is less than about 2 times the interval between digitisation levels of the digitiser, more preferably less than about the interval between digitisation levels of the digitiser, still more preferably less than about 75% of the interval between digitisation levels of the digitiser. Thus, the total noise present in the signal or signals is preferably between about 25% of the interval between digitisation levels of the digitiser and 2 times the interval between digitisation levels of the digitiser, more preferably between about 25% of the interval between digitisation levels of the digitiser and the interval between digitisation levels of the digitiser, still more preferably between about 25% of the interval between digitisation levels of the digitiser and about 75% of the interval between digitisation levels of the digitiser.

Most preferably, the total noise present in the signal or signals is about 50% of the interval between digitisation levels of the digitiser.

In a preferred embodiment, the combination of detector noise and system noise (i.e. the "local" noise) is controlled to be greater than about 25% of the interval between digitisation levels of the digitiser, and the environmental noise is not controlled in this manner. Controlling (only) the detector noise and system noise (and not the environmental noise) in this way has a number of advantages.

Firstly, detector noise and system noise (local noise) can be controlled relatively easily and relatively more accurately, e.g. by choice of detector, system components, etc. In comparison, control of environmental noise typically requires complex and expensive arrangements such as shielding, etc., and unexpected sources of environmental noise may exist which cannot be accounted for and controlled.

Secondly, detector noise and system noise (local noise) will typically have a relatively "cleaner" spectrum when compared with environmental noise, e.g. may comprise or may approximate to white noise. This simplifies the signal processing required to obtain the final output signal, and ensures that the noise present in the system contains relatively little or no structure that could otherwise interfere with the periodic signal of interest.

Thus, the magnetometer system is arranged such that detector noise and system noise (the "local" noise) in each signal provided to the digitiser is greater than about 25% of the interval between digitisation levels of the digitiser.

Correspondingly, in a preferred embodiment, the magnetometer system is arranged such that noise in each signal comprises or approximates to white noise.

In a preferred embodiment, the local noise present in the signal or signals is less than about 2 times the interval between digitisation levels of the digitiser, more preferably less than about the interval between digitisation levels of the digitiser, still more preferably less than about 75% of the interval between digitisation levels of the digitiser. Thus, the local noise present in the signal or signals is preferably between about 25% of the interval between digitisation levels of the digitiser and 2 times the interval between digitisation levels of the digitiser, more preferably between about 25% of the interval between digitisation levels of the digitiser and the interval between digitisation levels of the digitiser, still more preferably between about 25% of the interval between digitisation levels of the digitiser and about 75% of the interval between digitisation levels of the digitiser.

Most preferably, the local noise present in the signal or signals is about 50% of the interval between digitisation levels of the digitiser.

The magnetometer system may be configured such that the (local) noise is greater than about 25% of the interval between digitisation levels of the digitiser in any manner as desired.

In one preferred embodiment, the or each detector is designed so as to produce at least some or all of the desired noise. This may be achieved as desired.

In this regard, the Applicants have recognised that the requirement for there to be a relatively large amount of noise in the magnetometer system of the present invention advantageously relaxes the design constraints on the detectors (e.g. coils). This then allows the detectors to be designed with less emphasis, e.g. on the signal to noise characteristics of the detectors (coils), and with relatively more emphasis on size, shape, and weight considerations in order to provide a magnetometer system that is more suitable for medical magnetometry (and in particular for magneto cardiography). Accordingly, the or each detector preferably comprises a planar coil, e.g. as described above.

Thus, at least some of the desired noise may be provided by detector noise such as shot noise, Johnson noise, etc.

At least some of the desired noise may be provided by system noise such as electronics noise, etc. System noise in this context may include sources of noise within the magnetometer system (e.g. the magnetometer electronics) that introduce noise to the signal before the signal is digitised by the digitiser.

In a particularly preferred embodiment, the resistance of the or each detector (e.g. coil or group of plural coils connected in series) is selected (controlled) so as to produce at least some or all of the desired noise.

In this regard, the Applicants have recognised that selecting (controlling) the resistance of the or each detector (e.g. coil(s)) is equivalent to selecting (controlling) the (local) noise. This is because both the detector noise, i.e. Johnson noise, and the system noise, i.e. voltage noise and current noise, increase monotonically as the detector (e.g. coil(s)) resistance increases. In other words, the local noise (detector noise and system noise) increases monotonically as the detector (e.g. coil(s)) resistance increases.

Thus, in a particularly preferred embodiment, the resistance of the or each detector (e.g. coil or group of plural coils connected in series) is configured such that the noise in each signal provided to the digitiser for digitising is greater than about 25% of the interval between digitisation levels of the digitiser.

The resistance of the or each detector (e.g. coil(s)) may be selected as appropriate to produce the desired noise. The Applicants have found, in particular, that the resistance of the or each detector (e.g. coil(s)) should be (and is preferably) at least 5 Ohms, more preferably at least 10 Ohms. Thus, preferably, the or each detector (e.g. coil or group of plural coils connected in series) is configured to have a resistance of $\geq$ 5 Ohms, more preferably $\geq$ 10 Ohms.

In these embodiments, the resistance of the or each detector (e.g. coil(s)) may take any value that is greater than about 5 Ohms. However, as described above, higher levels of noise (higher resistances) may mean that more periods of the signal must be averaged in order to obtain an output signal with a useful information content. This in turn may mean that a longer signal acquisition period is required. Excessively long acquisition periods may, however, be undesirable in practical medical situations.

In this regard, the Applicants have found that the maximum acquisition time that a patient can tolerate, e.g. in a medical setting, is around 15 minutes. As such, the acquisition time should be (and is preferably) less than 15 minutes, preferably less than 10 minutes, more preferably less than 5 minutes, and more preferably less than 3 minutes.

Limiting the acquisition time in this manner limits the maximum (local) noise that the system should be configured to have, and accordingly limits the maximum resistance that the or each detector (e.g. coil(s)) should be configured to have.

The Applicants have found, in particular, that the resistance of the or each detector (e.g. coil(s)) should be (and is preferably) less than 3000 Ohms (i.e. to ensure that the required acquisition time is less than around 15 minutes). Thus, preferably, the or each detector (e.g. coil or group of plural coils connected in series) is configured to have a resistance of $\leq$ 3000 Ohms, preferably $\leq$ 2000 Ohms, more preferably $\leq$ 1000 Ohms, more preferably $\leq$ 500 Ohms, and still more preferably $\leq$ 200 Ohms.

Thus, in a particularly preferred embodiment, the or each detector (e.g. coil or group of plural coils connected in series) is configured to have a resistance in the range 5 to 3000 Ohms, and most preferably in the range 10 to 200 Ohms.

Correspondingly, in an embodiment, each detector comprises a coil or plural coils connected in series, wherein each detector has a maximum outer diameter of 7 cm or less, and a configuration such that the resistance of the coil or the plural coils connected in series is in the range 5 to 3000 Ohms.

In these embodiments, the or each detector (e.g. coil(s)) may be configured to have the desired resistance in any suitable manner. For example, the type and/or design of detector (e.g. induction coil and/or planar coil), the number of turns on the or each coil, the number of coils (e.g. connected in series), and/or the resistance per unit length (e.g. the cross-sectional area) of the wire, etc., may be selected as desired in order to produce the desired (local) noise.

Other arrangements would be possible.

In another preferred embodiment, one or more additional sources of noise may be provided in the system. For example, a noise generator may be provided and used to provide at least some of the desired noise. This allows for a greater degree of control over the level of noise present in the system. This may also allow the noise to more closely approximate to white noise.

Thus, in a preferred embodiment, the magnetometer system comprises a noise generator for adding noise into each signal. Equally, in a preferred embodiment, the method comprises adding noise into each signal.

The noise generator should (and does preferably) add noise to the signal prior to digitisation. In a preferred embodiment, the noise generator is provided as part of the system electronics, prior to (upstream of) the ADC. The noise generator may be provided prior to (upstream of) the amplifier or after (downstream of) the amplifier.

In this regard, the Applicants have recognised that in practice it may be possible for any inherent "system" noise and any environmental noise to be less than about 25% of the interval between digitisation levels in use, for example where environmental noise is particularly small and/or is otherwise removed from the detector signal(s) and/or depending on the design of the detector(s), etc., and that this would result in a poor output signal (for the reasons given above). Accordingly, there is a requirement to ensure that there is "sufficient" noise in the signal(s) that is digitised. This may be achieved by appropriate design of the magnetometer system, detector(s), etc., and/or a noise generator may be used to increase the noise to greater than about 25 % of the digitisation interval.

In a preferred embodiment, the level (amplitude) of the noise generated by the noise generator (the amount of "added" noise) is controllable (and is preferably controlled), e.g. to allow the system to be optimised as desired.

The amount of noise that is added to the signal by the noise generator may be manually (e.g. by means of a dial or other device) or automatically controlled (e.g. by means of software or otherwise).

Equally, the amount of noise that is added to the signal by the noise generator may be manually or automatically adjusted (varied), e.g. optimised.

For example, in one preferred embodiment the amount of added noise may be adjusted (e.g. optimised) by (manually or automatically) varying the amount of added noise, and (manually or automatically) monitoring the effects of the added noise on the recovered signal, e.g. until a desired or suitable, e.g. optimised, (output) signal is provided.

In another preferred embodiment, the amount of added noise may be (manually or automatically) selected (and preferably optimised) by measuring the "natural" noise in the system, and then controlling the "added" noise in response to the measurement, e.g. and preferably so as to ensure that the noise in each signal is greater than about 25% of the interval between digitisation levels of the digitiser.

The system noise (or "natural" noise) can be measured, e.g. by switching the detector at the input of the amplifier out of circuit, and terminating the input with an equivalent impedance, and then, e.g., either directly determining the number of noise bits on the digitiser (analogue to digital converter) or adding noise until the measured system noise is some multiple (e.g. two times) of the initial measurement. For example, where noise is added until the measured system noise is two times the initial measurement, then the added noise will be equal to the system noise.

In these embodiments, the amount of added noise may be controlled and/or optimised as part of the initialisation of the magnetometer system, e.g. automatically as part of an initialisation routine for the magnetometer system.

The noise generator may comprise, for example, a reverse biased Zener diode e.g. configured to generate white noise, optionally together with an amplifier, e.g. configured to amplify the output from the reverse biased Zener diode to achieve the required noise amplitude.

Alternatively, the noise generator may comprise a shift register arrangement, e.g. an arrangement of shift registers preferably with feedback. This may be implemented, e.g. using field programmable gate arrays.

Other arrangements would, of course, be possible.

In a preferred embodiment, one or more steps are taken to eliminate and/or compensate for any environmental noise or magnetic field interference that may exist in the signal(s) prior to digitisation. Any suitable such techniques may be used, although it should be noted here that the present invention does not require the use of a magnetically shielded environment.

In a particularly preferred embodiment, the mains (line) frequency (50 Hz in the UK) is removed from the signal, preferably by using an appropriate filter, such as and preferably a notch filter, on the signal prior to digitisation. The Applicants have found that using a filter tuned to the line frequency is sufficient to eliminate most environmental noise from the signal. In a preferred embodiment, a low pass filter with an appropriate cut-off frequency (e.g. 40 Hz, where the line frequency is 50 Hz) is used additionally to (try to) remove any remaining high frequency environmental noise.

Thus, in a particularly preferred embodiment, the method of the present invention further comprises removing the mains (line) frequency from the signal prior to digitisation, preferably by applying an appropriate filter, preferably a notch filter, to the signal. Most preferably the signal for analysis is also low-pass filtered to try to remove any remaining high frequency environmental noise.

Similarly, the magnetometer system of the present invention preferably further comprises an appropriate filter, such as and preferably a notch filter, set to the mains (line) frequency that acts on the signal(s) from the detector(s), most preferably together with a further low pass filter that acts on the signal of the mains (line) frequency filter.

In a preferred embodiment, the magnetometer system comprises a low impedance amplifier connected to the ends of the or each detector (coil), which amplifier is then connected to a low pass filter, e.g. with a frequency cut-off of 250 Hz, and a notch filter to remove line noise (e.g. 50 Hz).

Other or further techniques to try to eliminate or compensate for the effects of background noise can be used if desired. One preferred suitable such technique is background field subtraction using a background field only pick up detector (coil) (i.e. a detector that is not sensitive to the local field of the subject), preferably in conjunction with appropriate coil matching (active or passive). The background field only pick up detector(s) should be, and preferably are, configured the same as the detector(s) that are being used to detect the "wanted" signal (the periodic signal of interest).

Thus in a preferred embodiment, the apparatus and method of the present invention uses background noise pickup subtraction, preferably with coil matching, to try to account for (and compensate for) the presence of background magnetic fields.

In this case, where the system uses plural detectors, one or more of the detectors could be used as background pickup detectors (i.e. to detect the background magnetic field, rather than the subject's magnetic field). In this case, where there is an array of detectors, one or more of the outer detectors (e.g.) could be used to detect the background magnetic field, and/or if two or more layers of detector arrays are provided, one of the layers (or certain detectors in one of the layers) could be used to detect the background magnetic field. Thus in a preferred embodiment, the system comprises an array of plural detectors, and one or more of the detectors are used to detect the background magnetic field, with the remaining detectors being used to detect the magnetic field of interest (e.g. the subject's magnetic field).

In one preferred embodiment, detector (coil) output signal matching is achieved by using two detectors (two coils) and adding a global field to both detectors (coils) and then using lock in amplification of the difference signal and feedback to an amplifier which controls the gain of one of the detectors (coils). This facilitates precision matching of the detectors without the need for precision manufacturing of the detectors (coils) (which can be very difficult and expensive). The frequency of the global modulation field is preferably significantly higher than the frequency that is needed for medical detection (10-60 Hz), so as to move the frequency for lock in detection well above the frequency that is needed for medical detection. In a preferred embodiment the global modulation field has a frequency of at least 1 kHz.

A further advantage of using such a global modulation field coil matching technique is that it can then be used to subtract all global interfering (noise) fields, not just the mains (line) noise.

It should be noted that the Applicants have found that heart beat scale sensitivity can be achieved with the present invention without using gradient or background noise subtraction (or any equivalent process to compensate for background noise), although using gradient or background noise subtraction (or an equivalent process) will allow a useful signal to be produced more quickly.

In a preferred embodiment, any remaining environmental noise (where present) may be reduced and/or removed in post processing.

The system and method of the present invention can be used as desired to analyse the magnetic field, e.g. of the subject's heart. Preferably, suitable measurements are taken to allow an appropriate magnetic scan image of the heart (or other body region of interest) to be generated, which image can then, e.g., be compared to reference images for diagnosis. The present invention can be used to carry out any known and suitable procedure for imaging the magnetic field of the heart.

Preferably 16 to 50 (or more) sampling positions (detection channels) are detected in order to generate the desired scan image.

The present invention accordingly extends to the use of the magnetometer system of the present invention for analysing, and preferably for imaging, the magnetic field generated by a subject's heart (or other body region), and to a method of analysing, and preferably of imaging, the magnetic field generated by a subject's heart (or other body region) comprising using the method or system of the present invention to analyse, and preferably to image, the magnetic field generated by a subject's heart (or other region of the body). The analysis, and preferably the generated image, is preferably used for diagnosis of (to diagnose) a medical condition, such as abnormality of the heart, etc.

Thus in an embodiment the method comprises:

using the averaged signal or signals to analyse the magnetic field generated by the region of the subject's body; and
using the analysis of the magnetic field generated by the region of the subject's body to diagnose said medical condition.

In this embodiment, the signal (features of interest) from the detector or detectors are preferably used to produce an image representative of the magnetic field generated by the region of the subject's body, and the method preferably then comprises comparing the image obtained with a reference image or images to diagnose the medical condition. The medical condition is, as discussed above, preferably one of: abnormality of the heart, a bladder condition, pre-term labour, foetal abnormalities or abnormality of the head or brain.

As will be appreciated by those skilled in the art, these embodiments of the present invention can and preferably do include any one or more or all of the preferred and optional features of the invention described herein, as appropriate.

As will be appreciated from the above, an advantage of the present invention is that it can be used in the normal hospital or surgery or other environment, without the need for magnetic shielding. Thus, in a particularly preferred embodiment, the methods of the present invention comprise using the magnetometer system to detect the magnetic field of a subject's heart (or other body region) in a non-magnetically shielded environment (and without the use of magnetic shielding).

As will be appreciated by those skilled in the art, all of the aspects and embodiments of the invention described herein can and preferably do include any one or more or all of the preferred and optional features of the present invention, as appropriate.

The methods in accordance with the present invention may be implemented at least partially using software e.g. computer programs. It will thus be seen that when viewed from further aspects the present invention provides computer software specifically adapted to carry out the methods herein described when installed on data processing means, a computer program element comprising computer software code portions for performing the methods herein described when the program element is run on data processing means, and a computer program comprising code means adapted to perform all the steps of a method or of the methods herein described when the program is run on a data processing system. The data processing system may be a microprocessor, a programmable FPGA (Field Programmable Gate Array), etc.

The invention also extends to a computer software carrier comprising such software which when used to operate a magnetometer system comprising data processing means causes in conjunction with said data processing means said system to carry out the steps of the methods of the present invention. Such a computer software carrier could be a physical storage medium such as a ROM chip, CD ROM or disk, or could be a signal such as an electronic signal over wires, an optical signal or a radio signal such as to a satellite or the like.

It will further be appreciated that not all steps of the methods of the invention need be carried out by computer software and thus from a further broad aspect the present invention provides computer software and such software installed on a computer software carrier for carrying out at least one of the steps of the methods set out herein.

The present invention may accordingly suitably be embodied as a computer program product for use with a computer system. Such an implementation may comprise a series of computer readable instructions either fixed on a tangible medium, such as a non-transitory computer readable medium, for example, diskette, CD ROM, ROM, or hard disk. It could also comprise a series of computer readable instructions transmittable to a computer system, via a modem or other interface device, over either a tangible medium, including but not limited to optical or analogue communications lines, or intangibly using wireless techniques, including but not limited to microwave, infrared or other transmission techniques. The series of computer readable instructions embodies all or part of the functionality previously described herein.

Those skilled in the art will appreciate that such computer readable instructions can be written in a number of programming languages for use with many computer architectures or operating systems. Further, such instructions may be stored using any memory technology, present or future, including but not limited to, semiconductor, magnetic, or optical, or transmitted using any communications technology, present or future, including but not limited to optical, infrared, or microwave. It is contemplated that such a computer program product may be distributed as a removable medium with accompanying printed or electronic documentation, for example, shrink wrapped software, pre-loaded with a computer system, for example, on a system ROM or fixed disk, or distributed from a server or electronic bulletin board over a network, for example, the Internet or World Wide Web.

A number of preferred embodiments of the present invention will now be described by way of example only and with reference to the accompanying drawings, in which:

Figure 1 shows schematically the use of an embodiment of the present invention for detecting the magnetic field of a subject's heart;

Figures 2-5 show further exemplary arrangements of the use of an embodiment of the present invention when detecting the magnetic field of a subject's heart;

Figure 6 shows schematically a coil arrangement in accordance with an embodiment of the present invention;

Figure 7 shows a further exemplary arrangement of the use of an embodiment of the present invention when detecting the magnetic field of a subject's heart;

Figure 8 shows a probability distribution;

Figure 9 shows Fisher information as a function of noise;

Figures 10 and 11 show schematically magnetometer systems in accordance with embodiments of the present invention;

Figure 12 shows schematically the operation of a reverse biased Zener diode in accordance with an embodiment of the present invention;

Figure 13 shows the output of various white noise generators;

Figures 14-19 show coil arrangements in accordance with embodiments of the present invention; and Figure 20 shows a plot of coil resistance versus noise voltage output of a magnetometer system configured in accordance with an embodiment of the present invention.

Like reference numerals are used for like components where appropriate in the Figures.

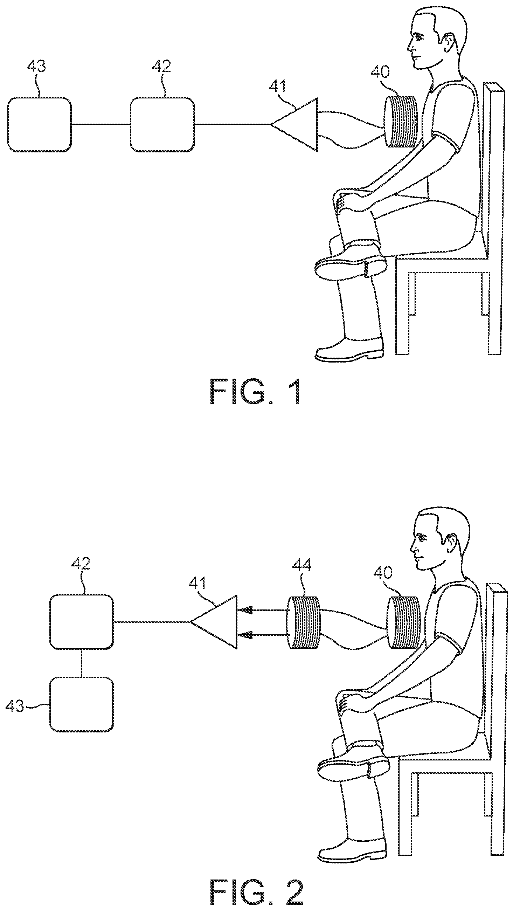

Figure 1 shows schematically the basic arrangement of a preferred embodiment of a magnetometer system that may be operated in accordance with the present invention. This magnetometer system is specifically intended for use as a cardiac magnetometer (for use to detect the magnetic field of a subject's heart). However, the same magnetometer design can be used to detect the magnetic field produced by other body regions, for example for detecting and diagnosing bladder conditions, pre-term labour, foetal abnormalities and for magnetoencephalography. Thus, although the present embodiment is described with particular reference to cardio-magnetometry, it should be noted that the present embodiment (and the present invention) extends to other medical uses as well.

The magnetometer system comprises a detector 40 coupled to a detection circuit 41 that may contain a number of components. The detector 40 may be an induction coil 40, e.g., as described further below.

The detection circuit 41 may comprise a low impedance pre amplifier, such as a microphone amplifier, that is connected to the coil 40, a low pass filter, e.g. with a frequency cut off of 250 Hz, and a notch filter to remove line noise (e.g. 50 Hz).

The current output from the coil 40 is processed and converted to a voltage by the detection circuit 41 and provided to an analogue to digital converter (ADC) 42 which digitises the analogue signal from the coil 40 and provides it to a data acquisition system 43.

A biological signal that is correlated to the heartbeat, e.g. an ECG or Pulse-Ox trigger from the test subject is used as a detection trigger for the digital signal acquisition, and the digitised signal over a number of trigger pulses is then binned into appropriate signal bins, and the signal bins overlaid or averaged, by the data acquisition unit 43.

The coil 40 and detection circuit 41 may be arranged such that the coil 40 and the preamplifier of the detection circuit 41 are arranged together in a sensor head or probe which is then joined by a wire to a processing circuit that comprises the remaining components of the detection circuit 41. Connecting the sensor head (probe) and the processing circuit by wire allows the processing circuit to be spaced from the sensor head (probe) in use.

With this magnetometer, the sensor head (probe) will be used as a magnetic probe by placing it in the vicinity of the magnetic fields of interest.

Figure 2 shows an improvement over the Figure 1 arrangement, which uses in particular the technique of gradient subtraction to try to compensate for background noise. In this case, an inverse coil 44 is used to attempt to subtract the effect of the background noise magnetic field from the signal detected by the probe coil 40. The inverse coil 44 will, as is known in the art, be equally sensitive to any background magnetic field, but only weakly sensitive to the subject's magnetic field. The inverse coil 44 can be accurately matched to the pick-up coil 40 by, for example, using a movable laminated core to tune the performance of the inverse coil to that of the pick-up coil 40.

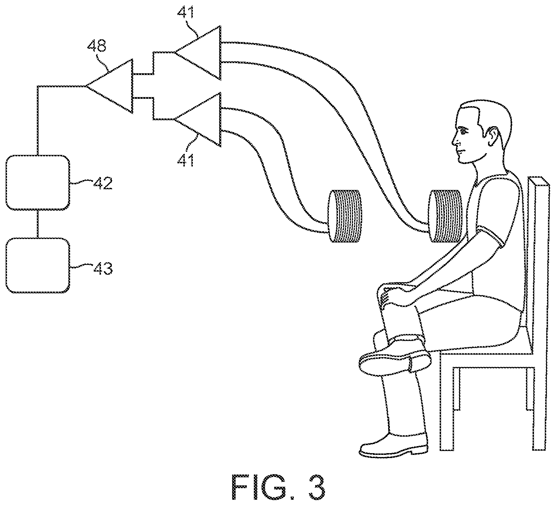

Figure 3 shows an alternative gradient subtraction arrangement. In this case, both coils 40, 44 have the same orientation, but their respective signals are subtracted using a differential amplifier 45. Again, the best operation is achieved by accurately matching the coils and the performance of the detection circuits 41. Again, a movable laminated core can be used to tune the performance of one coil to match the performance of the other.

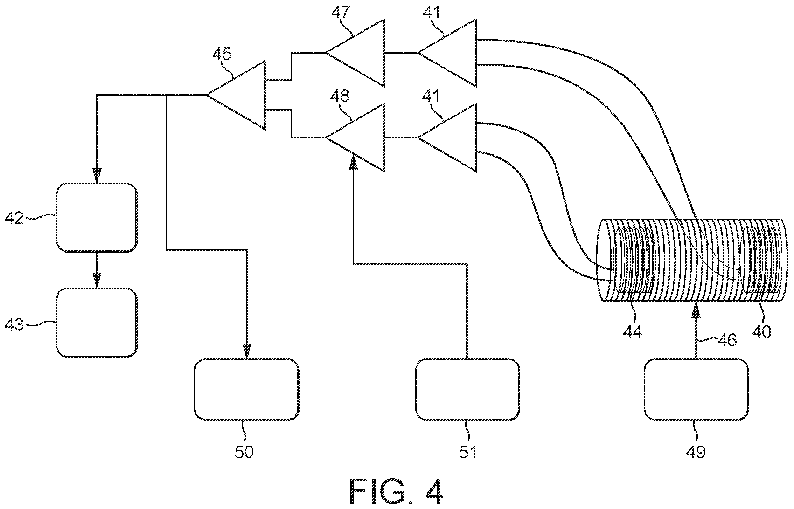

Figure 4 shows a further preferred arrangement. This circuit operates on the same principle as the arrangement of Figure 3, but uses a more sophisticated method of field cancellation, and passive coil matching. In particular, a known global magnetic field 44 is introduced to both coils 40, 44 to try to remove background magnetic field interference.

In this circuit, the outputs from the detection circuits 41 are passed through respective amplifiers 47, 48, respectively, before being provided to the differential amplifier 45. At least one of the amplifiers 47, 48 is tuneable. In use, a known global field 46, such as 50 Hz line noise, or a signal, such as a 1 kHz signal, applied by a signal generator 49, is introduced to both coils 40, 44. The presence of a signal on this frequency on the output of the differential amplifier 45, which can be observed, for example, using an oscilloscope 50, will then indicate that the coils 40, 44 are not matched. An amplifier control 51 can then be used to tune the tuneable voltage controlled amplifier 48 to eliminate the global noise on the output of the differential amplifier 45 thereby matching the outputs from the two coils appropriately.

Most preferably in this arrangement, a known global field of 1 kHz or so is applied to both coils, so as to achieve the appropriate coil matching for the gradient subtraction, but also a filter to remove 50 Hz noise is applied to the output signal.

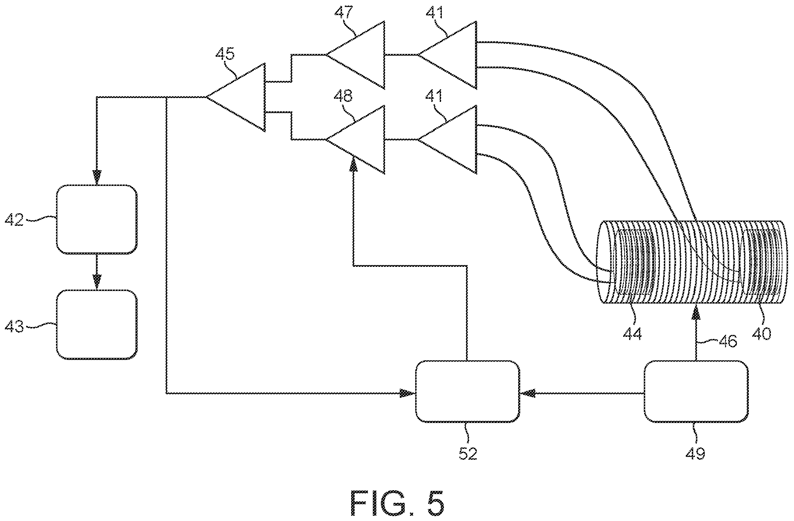

Figure 5 shows a further variation on the Figure 4 arrangement, but in this case using active coil matching. Thus, in this arrangement, the outputs of the coils 40, 44 are again channelled to appropriate detection circuits 41, and then to respective amplifiers 47, 48, at least one of which is tuneable. However, the tuneable amplifier 48 is tuned in this arrangement to remove the common mode noise using a lock-in amplifier 52 or similar voltage controller that is appropriately coupled to the output from the differential amplifier 45 and the signal generator 49.

The above embodiments of the present invention show arrangements in which there is a single pickup coil that may be used to detect the magnetic field of the subject's heart. In these arrangements, in order then to make a diagnostic scan of the magnetic fields generated by a subject's heart, the single pickup coil can be moved appropriately over the subject's chest to take readings at appropriate spatial positions over the subject's chest. The readings can then be collected and used to compile appropriate magnetic field scans of the subject's heart.

It would also be possible to arrange a plurality of coil and detection circuit arrangements, e.g. of the form shown in Figure 1, in an array, and to then use such an array to take measurements of the magnetic field generated by a subject's heart. In this case, the array of coils could be used to take readings from plural positions over a subject's chest simultaneously, thereby, e.g., avoiding or reducing the need to take readings using the same coil at different positions over the subject's chest.

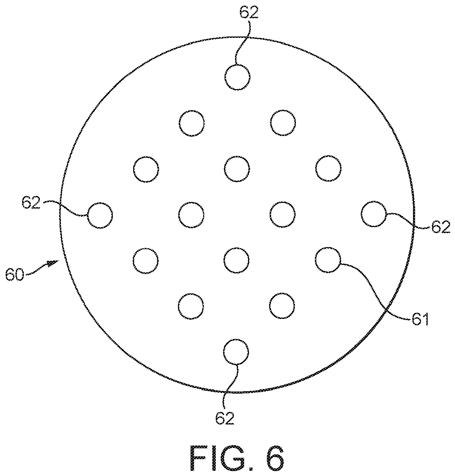

Figure 6 shows a suitable coil array arrangement that has an array 60 of 16 detection coils 61, which may be then placed over a subject's chest to measure the magnetic field of a subject's heart at 16 sampling positions over the subject's chest. In this case, each coil 61 of the array 60 should be configured as described above and connected to its own respective detection circuit (i.e. each individual coil 61 will be arranged and have a detection circuit connected to it as shown in Figure 1). The output signals from the respective coils 61 can then be combined and used appropriately to generate a magnetic scan of the subject's heart.

Other array arrangements could be used, if desired, such as circular arrays, irregular arrays, etc.

More (or less) coils could be provided in the array, e.g. up to 50 coils, or more than 50 coils. For example, where it is desired to measure the magnetic field of a different region of a subject's body (i.e. other than the heart), then an increased number of coils may be provided so as to provide an appropriate number of sampling points and an appropriate spatial coverage for the region of the subject's body in question.

It would also be possible in this arrangement to use some of the coils 61 to detect the background magnetic field for the purposes of background noise subtraction, rather than for detecting the wanted field of the subject's heart. For example, the outer coils 62 of the array could be used as background field detectors, with the signals detected by those coils then being subtracted appropriately from the signals detected by the remaining coils of the array. Other arrangements for background noise subtraction would, of course, be possible.

It would also be possible to have multiple layers of arrays of the form shown in Figure 6, if desired. In this case, there could, for example, be two such arrays, one on top of each other, with the array that is closer to the subject's chest being used to detect the magnetic field generated by the subject's heart, and the array that is further away being used for the purposes of background noise detection.

To measure the magnetic fields generated by the heart, the above arrangements can be used to compile magnetic field scans of a subject's heart by collecting magnetic field measurements at intervals over the subject's chest. False colour images, for example, can then be compiled for any section of the heartbeat, and the scans then used, for example by comparison with known reference images, to diagnose various cardiac conditions. Moreover this can be done for significantly lower costs both in terms of installation and on-going running costs, than existing cardiac magnetometry devices.

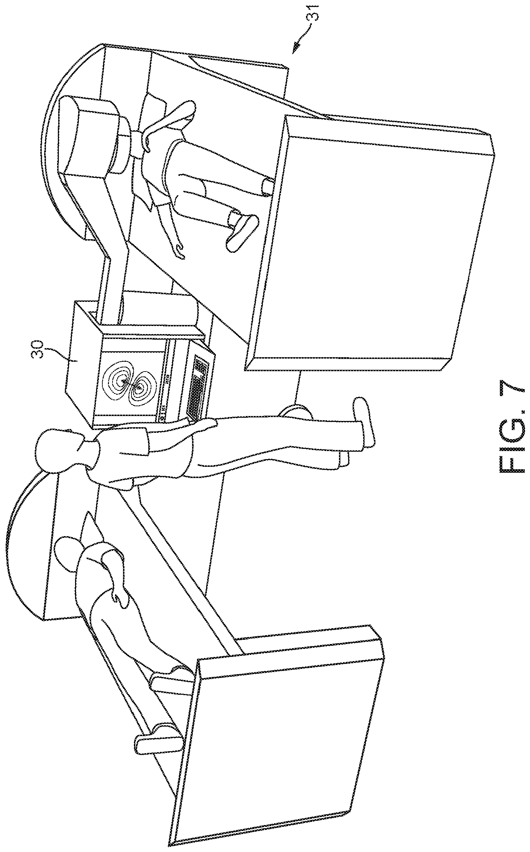

Figure 7 shows an exemplary arrangement of the magnetometer as it is envisaged it may be used in a hospital, for example. The magnetometer 30 is a portable device that may be wheeled to a patient's bedside 31 where it is then used to take a scan of the patient's heart (e.g.). There is no need for any magnetic shielding, cryogenic cooling, etc. The magnetometer 30 can be used in the normal ward environment.

It should be noted here that the signal generated by the pick-up coil in the present embodiments (and invention) will be the derivative of the useful signal, so the output signal can be (and preferably is) integrated over time to generate the wanted, useful signal. Such integration will also have the effect of tending to remove the effect of noise from the signal (provided the noise amplitude is not too big). Furthermore, the noise will remain in the integrated signal and so can be recovered if desired or needed, by taking the derivative of the integrated signal.

The magnetometer system can be used in an analogous manner to detect and analyse other medically useful magnetic fields produced by other regions of the body, such as the bladder, head, brain, a foetus, etc.

In these embodiments, the bio-physical magnetic fields of interest are typically very small, and can be so small that they are significantly below spurious background noise signals. The conventional approach to detect these signals would be to attempt to remove the background using passive shielding and/or active cancellation.

However, the Applicants have recognised that at least some noise is actually desirable, and in particular that a noisy signal can increase the sensitivity of the apparatus to sub-threshold signals when attempting to recover very small periodic signals.

In the present embodiments, noise is used to enable sub-threshold signals to be detected. Sub-threshold in this sense refers to signals with a voltage amplitude that is smaller than the smallest voltage separation in the analogue to digital conversion (ADC) system 42. Such signals appear as a single structureless output with zero information content. However, noise can be used to increase the effective peak-to-peak voltage of the signal, triggering transitions in the digital conversion. Hence the noise allows a signal to be detected with positive information content.

To detect the repeating signal, a trigger function is used to inform the detection apparatus of the presence of the signal. The underlying target signal is acquired repeatedly, and signal averaging techniques are applied to the data.

The detected voltage will comprise two basic elements: a detected field that includes both the background and target fields, and an additional noise element.

The noise element has a number of potential sources, including thermal noise, Johnson noise and intensity noise. Such noise sources are unavoidable and will therefore be present on any detection system. This noise will be random and incoherent (and therefore uncorrelated) both spatially (between detectors) and as a function of time (for a single detector). Any noise component that does not conform to this model (such as environmental noise) can be dealt with in post processing of the data.

The processing of the signal involves the detection of the signal as a voltage that is digitised into a set of discrete levels. These levels are indexed by an integer value, denoted by x, referring to the strength, size or level of the signal with maximum level, X. If the width of the digitized levels is *v*, which represents the minimum detectable voltage movement, the maximum signal that can be detected is *V = Xv.*

Digitisation of the signal results in the loss of information. If the magnitude of the total signal S for any is less than *v*, then digitisation will result in a complete loss of information. The Fisher information is a way of measuring the amount of information that an observable random variable x carries about an unknown parameter Q upon which x depends.

The total signal to be detected is a function of two elements, the biomagnetic signal Q and environmental noise $\sigma$, giving a signal *S(Q, $\sigma$).* The extent to which x reveals information about Q, is determined by the degree to which the distributions of Q and $\sigma$ can be distinguished.

To determine the extent to which x can be used to measure Q, the Fisher information, *F(x, Q)*, is used, which is a measurement of the amount of information that an observable x carries about the otherwise unknown parameter Q upon which the probability of x depends.

In other words, a change in the signal S can produce a commensurate change in x, thereby changing the information. The extent to which this occurs (how much of a change in the Q is required to change x) determines the information that x contains. If *v* is larger than $\sigma$ then x is unlikely to change and therefore carries very little information, if x changes frequently then a single sample of x contains more information.

If *f(x, Q)* is the likelihood of x with respect to Q, then the Fisher information *F(x, Q)* is given by:

$$F(x,Q) = \int \left(\frac{d}{dQ}\log(f(x,Q))\right)^2 f(x,Q)\,dx,$$

where the likelihood function, *f(x, Q) = P(x|Q, $\sigma$)*, is given by the distribution of likely values that x can have for a given Q and noise level $\sigma$.

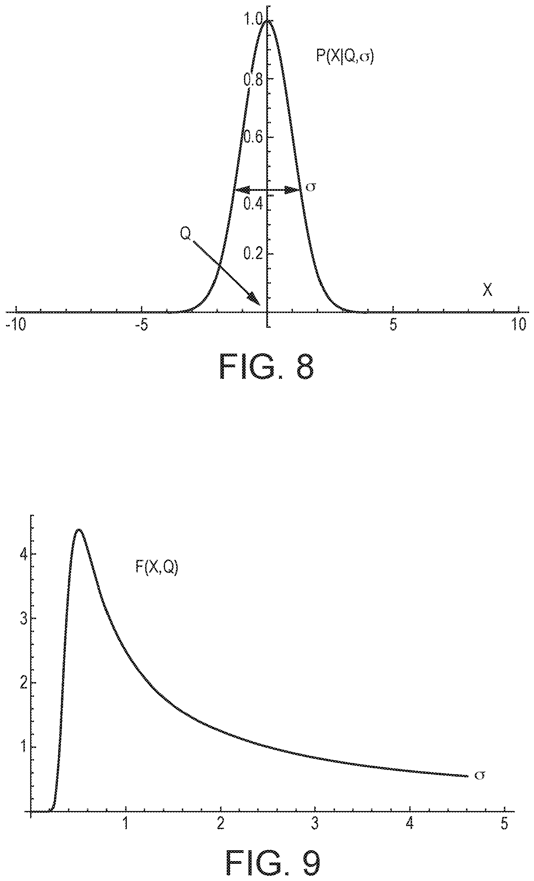

Figure 8 shows the probability distribution for x, which is determined by Q and the noise level.

The final component of the signal that must be considered is the impact of noise. For this example, the noise is considered to take the form of a small displacement:

0 < Q << *v,*

which is accompanied by random (incoherent) Gaussian white noise displacements. Under these conditions the conditional probability distribution for x takes the form:

$$P(x|Q,\sigma) = A\,Exp[-(x-Q)^2/(2\sigma^2)],$$

where A is a normalisation amplitude and the noise amplitude $\sigma$ is the width of a Gaussian distribution centered at Q.

In this case the Fisher information becomes:

$$F(x,Q) = \int \frac{Exp\left(-\frac{(x-Q)^2}{2\sigma^2}\right)(x-Q)^2}{\sigma^4}\,dx.$$

Figure 9 shows the Fisher information as a function of the noise level $\sigma$. As can be seen from Figure 9, below a threshold of noise amplitude, $\sigma$, the information drops to zero and it is impossible to extract information about Q from S. Hence, noise is in fact essential to extract the signal. Moreover, and counter-intuitively, there is no maximum noise level beyond which it is impossible to extract Q.

The Fisher information peaks when $\sigma \approx \frac{v}{2}$ and then falls monotonically, however it always remains above zero. Hence, the amount of information that can be extracted per iteration reduces, and more averaging is required to calculate an accurate value for Q. However, importantly it does not return to zero and therefore it is possible to determine Q for even large values of noise.

Thus, in the present embodiment, the magnetometer system is configured such that the noise in the signal (i.e. the noise amplitude of the signal or, where the noise comprises white noise or other Gaussian noise, the standard deviation of the noise amplitude of the signal) provided (input) to the digitiser is greater than about 25% of the interval between digitisation levels of the ADC, e.g. about 50% of the interval between digitisation levels of the ADC.

This solves the problem of maintaining a significantly large dynamic range in the presence of a large noise. It is possible to retain information at the small signal end while also capturing the full deviation of the noise. This shows that in fact, in the presence of noise it is possible to do away with low level sensitivity as long as the noise is sufficiently high.

Hence a smaller induction coil that is insufficiently sensitive to detect bio-magnetic fields in a low noise environment can be used in a higher noise environment to detect the same small bio-magnetic signals.

In order to extract data from the signal, a trigger is used to gate the coil output, and the signal is split into a number of gate cycles. Power-line and other noise background noise sources can be removed by filtering if required, but Johnson shot noise and other noise sources are retained to increase the dynamic range. The sub-signals are averaged to produce the output.

As each trigger instance delivers a small amount of information, this final step of averaging is used to deliver the final information content of the signal. Fine detail and small elements of the signal can be future extracted with more averaging.

Accordingly, in the present embodiment a signal that is smaller than the interval between digitisation levels of the digitiser, i.e. a signal that would otherwise be smaller than the minimum signal detectable by the digitiser can be detected. Similarly, a signal that is smaller than the noise, i.e. a signal with a noise ratio less than one, can be detected.

In particular, the P wave, QRS wave and/or T wave of the time varying magnetic field of a subject's heart or other signal features of interest, that is or are smaller than the interval between digitisation levels, can be detected.

The signal to noise ratio for the magnetometer system of the present embodiment for a "typical" heart scan conducted in a "typical" noise environment may be around -120dB (although it should be noted that the noise environment may be subject to significant variation). The signal to noise ratio for the system when the electronics system is terminated with a representative impedance (i.e. so as to exclude external environmental noise) may be around -55dB. The signal level amplitude of signal features of interest is significantly lower than the quantisation level of the ADC.

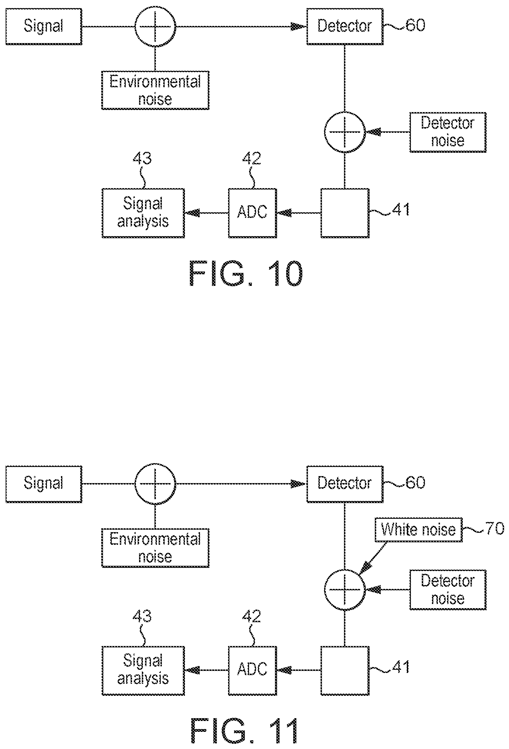

As shown in Figure 10, potential sources of noise in the magnetometer system according to the present embodiment include environmental noise, detector noise, and other system noise such as electronics noise, etc.

In the present embodiment, the combination of detector noise and system noise (i.e. the "local" noise) is controlled to be greater than about 25% of the interval between digitisation levels of the digitiser after the amplifier 41 and prior to the digitiser 42 (e.g. about 50% of the interval between digitisation levels of the digitiser), and the environmental noise is not controlled in this manner. In the present embodiment, most of the environmental noise is removed by using a notch filter to remove the mains (line) frequency (50 Hz in the UK) from the signal prior to digitisation. A low pass filter with an appropriate cut-off frequency (e.g. 40 Hz, where the line frequency is 50 Hz) may additionally be used to remove any remaining high frequency environmental noise.

This has a number of advantages. Firstly, detector noise and system noise (local noise) can be controlled relatively easily and relatively more accurately, e.g. by choice of detector, system components, etc. In comparison, control of environmental noise typically requires complex and expensive arrangements such as shielding, etc., and unexpected sources of environmental noise may exist which cannot be accounted for and controlled.

Secondly, detector noise and system noise (local noise) will typically have a relatively "cleaner" spectrum when compared with environmental noise, e.g. may comprise or may approximate to white noise. This simplifies the signal processing required to obtain the final output signal, and ensures that the noise present in the system contains relatively little or no structure that could otherwise interfere with the periodic signal of interest.

The desired amount of noise in the system can be provided in a number of different ways.

For example, the detector system can be designed in order to provide sufficient noise. In this case, at least some of the desired noise may be provided by detector noise such as shot noise, Johnson noise etc., and/or at least some of the desired noise may be provided by system noise such as electronics noise, etc. System noise in this context includes sources of noise within the magnetometer system (e.g. the magnetometer electronics) that introduce noise to the signal before the signal is digitised by the digitiser.

This advantageously relaxes the design constraints on the coils, and allows, e.g. the detectors to be designed with less emphasis on the signal to noise characteristics of the coils, and with relatively more emphasis on size, shape, and weight considerations in order to provide a magnetometer system that is more suitable for medical magnetometry (and in particular for magneto cardiography).

Additionally or alternatively, noise can be added to the system. Figure 11 shows one such embodiment, in which a white noise generator 70 is used to add noise to the signal. This allows for a greater degree of control over the level of noise present in the system, and may allow the noise to more closely approximate to white noise.

In this regard, the Applicants have recognised that in practice it may be possible for any inherent "system" noise and any environmental noise to fall below about 25% of the interval between digitisation levels in use, for example where environmental noise is particularly small and/or is otherwise removed from the detector signal(s) and/or depending on the design of the detector(s), etc., and that this would result in a poor output signal (for the reasons given above). Accordingly, in order to ensure that there is "sufficient" noise in the signal(s) that is digitised, the magnetometer system may be appropriately designed, and/or a noise generator may be used to increase the noise to greater than about 25 % of the digitisation interval.

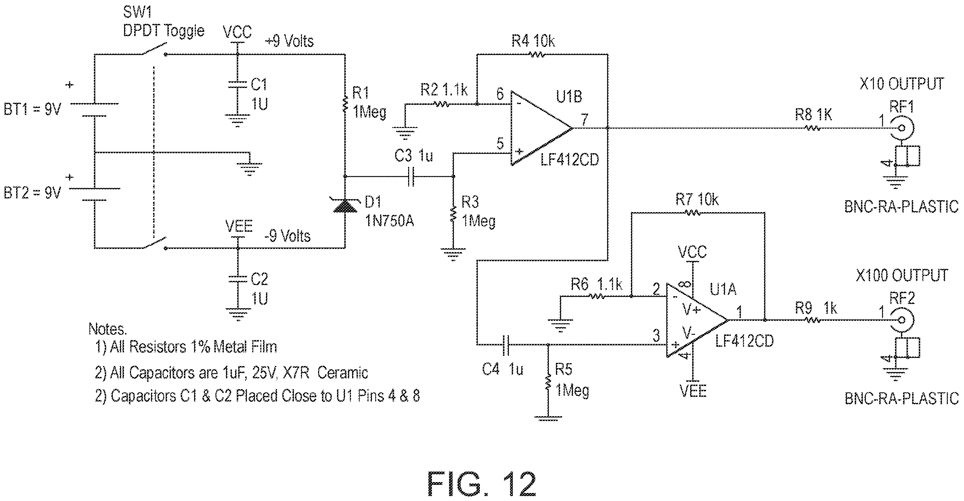

As shown in Figure 12, white noise can be generated by reverse biasing a Zener diode and amplifying the output to achieve the required amplitude.

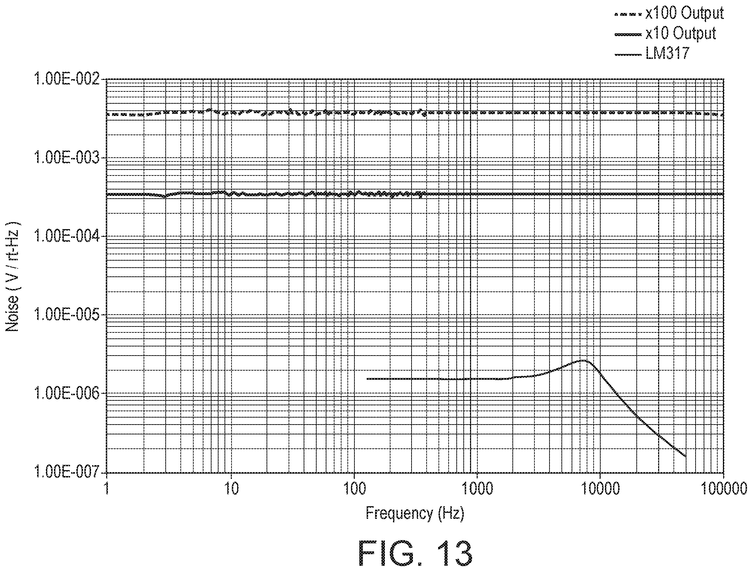

Alternative means of generating white noise that may be used include digital techniques such as a shift register arrangement using an arrangement of shift registers with feedback. Such designs may be implemented using field programmable gate arrays. Figure 13 shows the output of three different white noise generators in accordance with various embodiments.

The amount of noise that is added to the signal by the noise generator 70 may be manually controlled and/or optimised, e.g. by means of a dial or other device, or automatically controlled and/or optimised, e.g. by means of software or otherwise. For example, the amount of added noise may be optimised by varying the amount of added noise, and monitoring the effects of the added noise on the recovered signal until an optimised signal is provided.

Additionally or alternatively, the amount of added noise may be optimised by measuring the "natural" noise in the system, and then controlling the "added" noise in response to the measurement.

In this case, the system noise (or "natural" noise) can be measured by switching the detector at the input of the amplifier out of circuit, and terminating the input with an equivalent impedance, and then either directly determining the number of noise bits on the ADC or adding noise until the measured system noise is two times the initial measurement, whereupon the added noise will be equal to the system noise.

The amount of noise may be controlled or optimised as part of the initialisation of the magnetometer system, e.g. automatically as part of an initialisation routine for the magnetometer system.

The design of the induction coil for use in the preferred embodiments of the present invention will now be described. The coil should have an overall size that permits spatial resolution suitable for magneto cardiography.

Johnson noise is normally considered to be the limit of sensitivity of a magnetic detection coil, and therefore conventional coil designs aim to optimise signal over Johnson noise. Designs optimising signal to noise that limit Johnson noise are beneficial when other environmental noise sources are limited. However, in any practical circumstances, most sources of environmental background noise are much larger than either the target signal or the Johnson noise. Hence, the design criteria limiting Johnson noise can be substantially relaxed, indeed, as described above, Poissonian distributed noise, such as Johnson noise actually plays a positive role in signal extraction.

The frequency of the relevant magnetic signals of the heart is between 1 Hz and 60 Hz. Thus, the coil of the present embodiments is designed to be sensitive to magnetic fields at these frequencies.

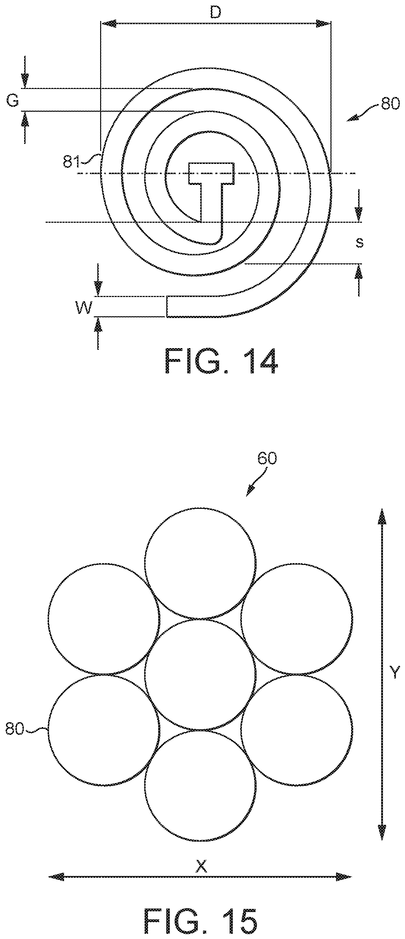

As shown in Figure 14, in the present embodiment, each coil comprises a planar coil 80, i.e., a coil with plural turns arranged in a single plane. Each planar coil 80 comprises a conductor track 81 arranged in a spiral. Any suitable conductor may be used for the planar coil, such as copper, gold, silver, carbon nanotubes, graphene, or similar.

Each planar coil 80 may have a maximum outer diameter D between 2 and 6 cm. The conductor track has width $W$ (a thickness in the direction parallel to the plane in which the planar coil's plural turns are arranged) of around 1 mm or less. For example, the width may be between around 0.1 and 1 mm, or between around 0.3 and 0.5 mm.

The turns of the planar coil are spaced apart (in the direction parallel to the plane in which the planar coil's plural turns are arranged) with a gap G of around 1 mm or less. For example, the spacing may be around 0.1 mm or less or 0.01 mm or less.

The conductor track is relatively flat in the direction orthogonal to the plane in which the coil's plural turns are arranged, and has a thickness Z (in the direction orthogonal to the plane in which the coil's plural turns are arranged) of around 0.2 mm or less. For example, the thickness Z may be around 0.1 mm or less, or around 0.05 mm or less. The thickness Z should not be less than around 0.035 mm.

The planar (spiral) coil may comprise desired number of turns, such as 2 or more turns.

Each planar coil 80 comprises a spiral conductor arranged on or within an electrically insulating substrate 82. The insulating substrate supports the conductor, and is accordingly substantially rigid. Any suitable insulator can be used for the insulating substrate, such as glass, plastic, reinforced plastic, etc. The substrate may also comprise a printed circuit board (PCB) material, such as FR4 and the like.

The Applicants have recognised that it can be beneficial to increase the width $W$ of the conductor track (its thickness in the direction parallel to the plane in which the planar coil's plural turns are arranged), and correspondingly to reduce the size of the gap G between each of the turns of the planar coil. This has the effect of reducing the resistance of the coil, while increasing the "useful" signal collecting area of the coil.

Increasing the height Z of the conductor track (its thickness in the direction orthogonal to the plane in which the coil's plural turns are arranged) also has the effect of reducing the resistance of the coil. However, this can affect the minimum spacing between turns that it is feasible to achieve in practice. For example, where chemical etching techniques are used to form the spiral track 81, increasing the conductor height can increase the minimum gap between each of the turns of the spiral that it is feasible to achieve in practice.

In one exemplary arrangement, the thickness Z is 0.035 mm, and the gap width G is 0.127 mm. Where the thickness Z is increased above around 0.05 mm, the track gap has to be increased. In another exemplary arrangement (e.g. using a 0.14 mm thick FR4 PCB), the thickness Z is 0.14 mm, and the gap width G is 0.25 mm. The Applicants have found that these value ranges are achievable in practice and provide planar coils that are capable of producing a useful output signal.

Laser etching may be used to achieve even smaller gap widths G between the turns of the planar coil, e.g. as small as 3-5 μm.

Each planar coil may optional further comprise a magnetic core. This has been found to improve performance of the planar coils. A magnetic core may be located within (at the axial centre of) each spiral planar coil, or otherwise adjacent to each spiral planar coil. Each planar spiral coil may have an inner diameter in the range 4-35 mm, or 15-22 mm, and correspondingly each magnetic core may have a diameter in the range 4-35 mm, or 15-22 mm.

The magnetic core should be made from a material with a high relative permeability such as a ferrite or other magnetic material. For example, the magnetic core may be made from a magnetic amorphous metal alloy and/or a nano-crystalline material. These materials can exhibit very high magnetic permeabilities, but can be lighter than other magnetic materials such as iron powder.

Nanocrystalline materials are poly-crystalline materials with very small grain sizes, the space between which is filled with amorphous materials.

Amorphous metals (sometimes referred to as metallic glasses or glassy metals) differ from traditional metallic materials and alloys in that they have highly disordered atomic structures instead of conventional crystalline or poly-crystalline lattices, and as such have a number or unique properties. They are typically produced from a mixture of differently sized metallic atoms which are quench-cooled at millions of degrees per second, removing the thermal energy for atoms to move and form ordered domains or grains. By alloying with certain magnetic materials such as iron, cobalt, and nickel, very high magnetic permeability and susceptibility materials are possible. Their high(er) resistance (similar to that of their molten components) reduces eddy current losses when subjected to alternating magnetic fields. Their low coercivity also reduces losses.

One exemplary such material is known as Met-Glas 2714a (Metallic Glass Alloy).

Utilising planar coils is beneficial, particular where as discussed above the magnetometer system comprises plural detectors arranged in plural layers, since this can result in a magnetometer system having a size, shape, and weight that is more suitable for medical magnetometry (and in particular for magneto cardiography). For example, Figure 15 shows a suitable coil array 60 arrangement that has an array of planar detection coils 80, which may be then placed over a subject's chest to measure the magnetic field of a subject's heart at 7 sampling positions over the subject's chest.

The or each array may comprise 30-45 planar coils, e.g. 37 planar coils, arranged in a two dimensional array. This number of planar coils has been found to provide particularly good spatial coverage for the human heart.

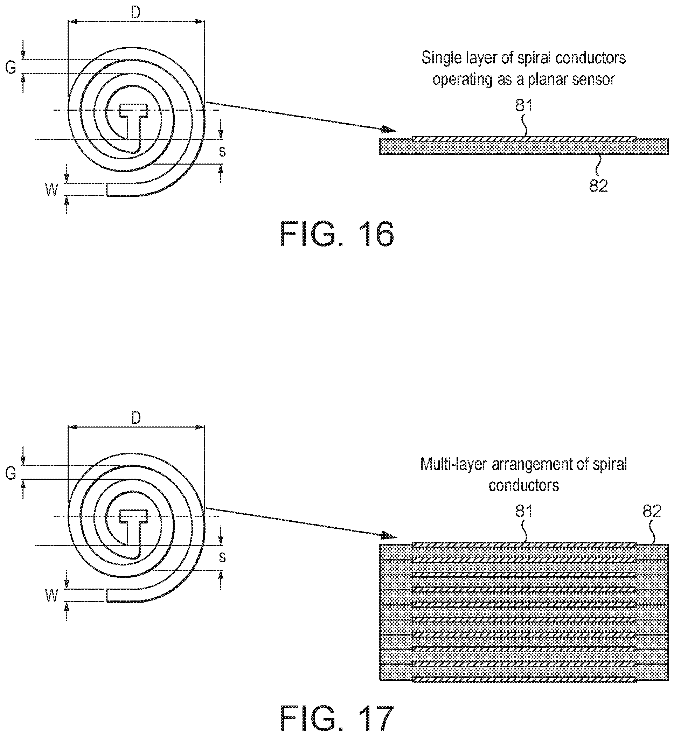

As shown in Figure 16, a single layer of planar coils may be utilised for measuring the magnetic field of a subject's heart. Alternatively, as shown in Figure 17, since the planar coils are relatively flat in the out-of-plane direction, plural layers of planar coils may be conveniently utilised for measuring the magnetic field of a subject's heart.

The one or more arrays of planar coils may comprise 20-120 layers of planar coils, e.g. 40-90 layers, one above the other. This has been found to result in a magnetometer system that produces a useful signal, while having a weight that is suitable for medical magnetometry (and in particular for magneto cardiography).

Each layer may be formed on its own PCB layer, or plural layers of planar coils may be arranged on or within a single multi-layer PCB, e.g. so as to reduce the overall weight of the magnetometer system.

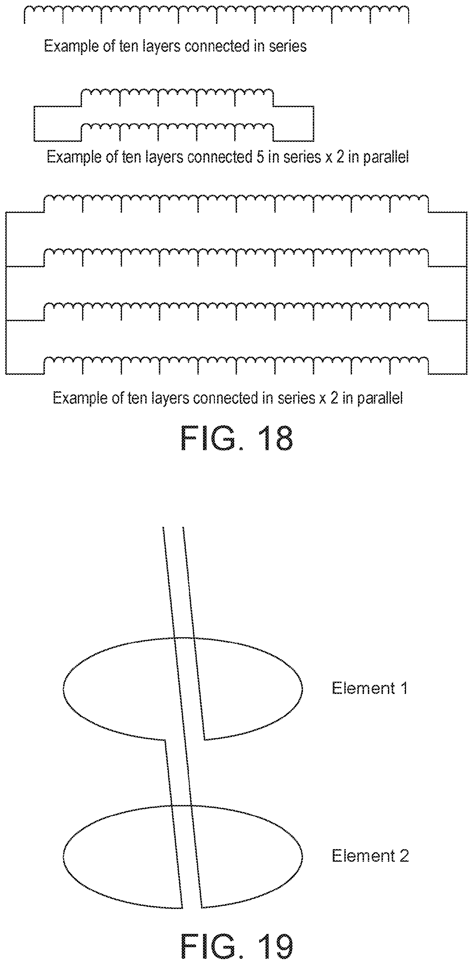

Where the magnetometer system comprises plural detectors, some or all of the detectors may be connected, e.g. in parallel and/or in series. Connecting plural detectors in series will have the effect of increasing the induced voltage for a given magnetic field strength. Connecting plural detectors in parallel will have the effect of reducing the thermal noise (Johnson noise) in the detectors. As shown in Figure 18, a combination of series and parallel connections can be used to optimise the balance of voltage and noise performance of the detectors.

As shown in Figure 19, one or more or each detector in the magnetometer system can be arranged in a gradiometer configuration, i.e. where two detectors are co-axially aligned (in the direction orthogonal to the plane in which each coil's windings are arranged), and where the signal from each of the coils is summed, e.g. to provide a measure of a change in the magnetic field in space.

As described above, according to the Fisher information, the presence of some noise allows small signals to be detected that would otherwise be undetectable. The Applicants have furthermore recognised that there is an optimum noise range that enables signal detection in a reasonable time.

As described above, the Fisher information is always positive, so that more noise always enhances small signals. However, the Fisher information reduces exponentially above a threshold of about 50% of the interval between digitisation levels of the digitiser. Higher levels of noise accordingly mean that more periods of the signal must be averaged in order to obtain an output signal with a useful information content. As such, an upper noise limit is set by the maximum practical scan time.

The Applicants have furthermore recognised that controlling the resistance of the coil or group of plural coils connected in series is equivalent to controlling the local noise. There are three main noise sources in the apparatus, namely (i) Johnson noise from the coil windings, (ii) current noise generated in the system, and (iii) circuit noise in the amplifier circuit. Noise sources (i) and (ii) are due to the induction coil and can be controlled for optimum noise performance.

Since adding more coils to the system increases the resistance, this can lead to the result that adding more coils to the sensor, in some cases, does not improve the system.

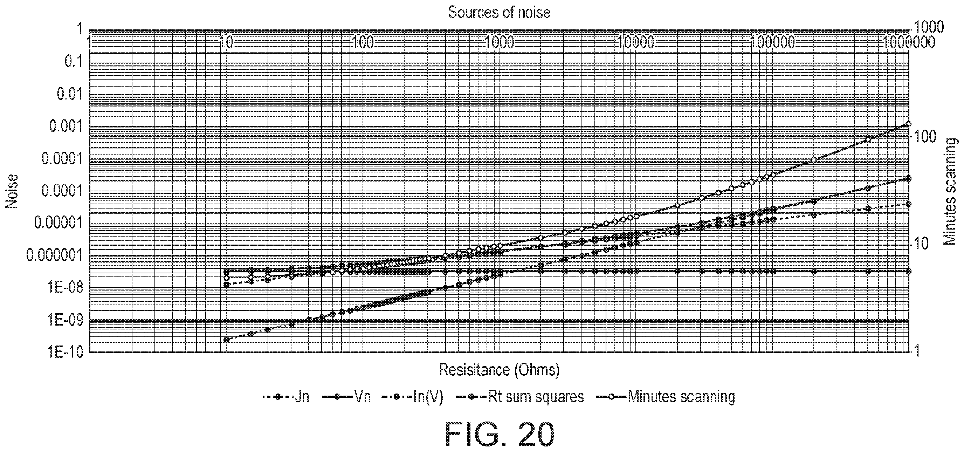

Figure 20 shows a plot of coil resistance against noise voltage output. As the number of turns on a coil increases so does the resistance of the coil. The resistance impacts Johnson noise, as well as the noise produced by the amplifier, i.e. in the form of voltage noise and current noise (this varies to some extent dependent on the circuit selected). Johnson noise remains the dominant source of noise.

There is an upper limit for the scan time that a patient can tolerate of around 10-15 minutes. Preferably this is less than 5 minutes and ideally less than 3 minutes. This time limit limits the maximum noise that the system can practically be configured to have, and in turn limits the maximum resistance that the coil or coils can practically be configured to have. In particular, the system should operate with resistance values that are to the left of the 15 minute point in Figure 20.

Accordingly, the optimum resistance range for the coil or coils is around 5-3000 Ohms, preferably 10-2000 Ohms, more preferably 10 to 200 Ohms.

The magnetometer system of the present embodiment can be configured to have a particular desired coil resistance by appropriate selection of the type and/or design of detector (e.g. induction coil and/or planar coil), the number of turns on the or each coil, the number of coils (e.g. connected in series), and/or the resistance per unit length (e.g. the cross-sectional area) of the wire, etc.

In one exemplary arrangement, each planar coil comprises a layer of a copper clad printed circuit board FR4 material. Each planar coil has a track width *W* of 0.1 mm and gap width G of 0.1 mm. The planar coil is in a spiral form with an inner diameter of 4 mm and an outer diameter 48 mm.

The PCB comprises a multi-layer PCB having ten layers of planar coils. Each of the individual layers is connected in series. The combined resistance for this arrangement is 400 Ohms. For an array of 37 planar coils with ten layers, the device can detect the magnetic field of a human heart with a scan time of around 1 hour.

Where a second ten layer PCB is added in series (a total of 20 layers), the device can detect the magnetic field of a human heart in 40 minutes. Where a third ten layer PCB is added in series (a total of 30 layers) the device can detect the magnetic field of a human heart in 15 minutes.

Where, however, additional layers are added in series, then the signal deteriorates. For example, a device having five ten layer PCBs (a total of 50 layers) requires >30 minutes to detect the magnetic field of a human heart. The resistance of this system is around 2000 Ohms.

In contrast, where thirty layers are placed in parallel with thirty layers (resistance 600 Ohms), the magnetic field of a human heart can be detected in 10 minutes. Where thirty layers are placed in parallel with thirty layers and again in parallel with thirty layers (resistance 400 Ohms) the magnetic field of a human heart can be detected in 5 minutes.

It will accordingly be appreciated that the amount of the noise in the system can be controlled by controlling the resistance of the system, which in turn can be controlled by selecting an appropriate combination of series and parallel connections between plural planar coils.

It can be seen from the above that the present invention, in its preferred embodiments at least, provides a magnetic imaging device that can be deployed effectively from both a medical and cost perspective in a wide range of clinical environments, e.g. for use when detecting magnetic fields generated by the heart. The magnetometer is, in particular, advantageous in terms of its cost, its practicality for use in clinical environments, and its ability to be rapidly deployed for near patient diagnosis and for a wide range of applications. It is non-contact, works through clothing, fast, compact and portable and affordable. An image can be recovered with high resolution after a minute of signal recording and absolute "single beat" sensitivity is potentially possible. Patient motion of up to 1-2 cm will not significantly degrade the image.

This is achieved, in the preferred embodiments of the present invention at least, by detecting, digitising and averaging plural repeating periods of the time varying magnetic field of a region of a subject's body overall plural periods, where the system is arranged such that the noise present in the signal to be digitised is greater than about 25% of the interval between digitisation levels of the digitiser.

**Claims**

1. A method of using a magnetometer system (30) to analyse the magnetic field of a region of a subject's body, the method comprising:

   using one or more detectors (60) to detect the time varying magnetic field of a region of a subject's body;
   using a digitiser (42) to digitise a signal or signals from the one or more detectors, each signal that is digitised including noise and a periodic signal produced by one or more of the one or more detectors due to the time varying magnetic field of the region of the subject's body; and
   averaging the digitised signal or signals over plural periods;
   wherein the magnetometer system is configured such that detector noise and system noise in each signal provided to the digitiser for digitisation is greater than about 25% of the interval between digitisation levels of the digitiser, such that the magnetometer system can detect one or more signal features of the periodic signal produced by one or more of the one or more detectors due to the time varying magnetic field of the region of the subject's body that are smaller than the interval between digitisation levels of the digitiser.

2. The method of claim 1, comprising:

using one or more arrays of plural detectors to detect the time varying magnetic field of a region of a subject's body.

3. The method of claim 1 or 2, comprising:
using plural layers of detectors to detect the time varying magnetic field of a region of a subject's body.

4. The method of claim 1, 2 or 3, comprising:

using plural detectors to detect the time varying magnetic field of a region of a subject's body;
wherein some or all of the detectors are connected in parallel and/or in series.

5. The method of any one of the preceding claims, comprising:

using plural detectors to detect the time varying magnetic field of a region of a subject's body;
wherein some or all of the detectors are arranged in a gradiometer configuration.

6. The method of any one of the preceding claims, wherein each detector comprises a planar coil.

7. The method of any one of the preceding claims, wherein the magnetometer system is configured such that detector noise and system noise in each signal provided to the digitiser for digitisation is less than about two times the interval between digitisation levels of the digitiser.

8. The method of any one of the preceding claims, wherein the magnetometer system is configured such that the detector noise and system noise in each signal comprises or approximates to white noise.

9. The method of any one of the preceding claims, wherein each detector comprises a coil or plural coils connected in series and has a configuration such that the resistance of the coil or the plural coils connected in series is in the range 5 to 3000 Ohms.

10. The method of any one of the preceding claims, further comprising using a noise generator to generate at least part of the noise in each signal.

11. The method of any one of the preceding claims, wherein the region of the subject's body whose magnetic field is being analysed comprises one of: the bladder, heart, head, brain, womb or a foetus.

12. A method of analysing the magnetic field of a subject's heart, the method comprising:
using the method of one of the preceding claims to analyse the time varying magnetic field of a subject's heart.

13. A magnetometer system (30) for medical use, comprising:

one or more detectors (60) for detecting the time varying magnetic field of a region of a subject's body;
a digitiser (42) configured to digitise a signal or signals from the one or more detectors, each signal that is digitised including noise and a periodic signal produced by one or more of the one or more detectors due to the time varying magnetic field of the region of the subject's body; and averaging circuitry configured to average the digitised signal or signals over plural periods;
wherein the magnetometer system is configured such that detector noise and system noise in each signal provided to the digitiser for digitisation is greater than about 25% of the interval between digitisation levels of the digitiser, such that the magnetometer system can detect one or more signal features of the periodic signal produced by one or more of the one or more detectors due to the time varying magnetic field of the region of the subject's body that are smaller than the interval between digitisation levels of the digitiser.

14. The magnetometer system of claim 13, wherein the magnetometer system is configured such that detector noise and system noise in each signal provided to the digitiser for digitisation is less than about two times the interval between digitisation levels of the digitiser.

15. The magnetometer system of claim 13 or 14, wherein each detector comprises a coil or plural coils connected in series and has a configuration such that the resistance of the coil or the plural coils connected in series is in the range 5 to 3000 Ohms.

**Patentansprüche**

1. Verfahren zum Verwenden eines Magnetometersystems (30), um das Magnetfeld eines Bereichs des Körpers eines Subjekts zu analysieren, wobei das Verfahren umfasst:

Verwenden eines oder mehrerer Detektoren (60), um das zeitlich veränderliche Magnetfeld eines Bereichs des Körpers eines Subjekts zu detektieren;
Verwenden eines Digitadunglisierers (42), um ein Signal oder Signale von dem einen oder den mehreren Detektoren zu digitalisieren, wobei jedes Signal, das digitalisiert wird, Rauschen und ein periodisches Signal einschließt, das von einem oder mehreren der einen oder mehreren Detektoren aufgrund des zeitlich veränderlichen Magnetfelds des Bereichs des Körpers des Subjekts erzeugt wird; und

Mittelwertbilden des digitalisierten Signals oder der digitalisierten Signale über eine Mehrzahl von Perioden;

wobei das Magnetometersystem so konfiguriert ist, dass das Detektorrauschen und Systemrauschen in jedem Signal, das dem Digitalisierer zur Digitalisierung bereitgestellt wird, größer als etwa 25 % des Intervalls zwischen Digitalisierungsniveaus des Digitalisierers ist, sodass das Magnetometersystem ein oder mehrere Signalmerkmale des periodischen Signals detektieren kann, das von einem oder mehreren des einen oder der mehreren Detektoren aufgrund des zeitlich veränderlichen Magnetfelds des Bereichs des Körpers des Subjekts erzeugt wird, die kleiner als das Intervall zwischen Digitalisierungsniveaus des Digitalisierers sind.

2. Verfahren nach Anspruch 1, umfassend:
Verwenden eines oder mehrerer Arrays aus einer Mehrzahl von Detektoren, um das zeitlich veränderliche Magnetfeld eines Bereichs des Körpers eines Subjekts zu detektieren.

3. Verfahren nach Anspruch 1 oder 2, umfassend:
Verwenden einer Mehrzahl von Detektorschichten, um das zeitlich veränderliche Magnetfeld eines Bereichs des Körpers eines Subjekts zu detektieren.

4. Verfahren nach Anspruch 1, 2 oder 3, umfassend:

Verwenden einer Mehrzahl von Detektoren, um das zeitlich veränderliche Magnetfeld eines Bereichs des Körpers eines Subjekts zu detektieren;
wobei einige oder alle der Detektoren parallel und/oder in Reihe geschaltet sind.

5. Verfahren nach einem der vorstehenden Ansprüche, umfassend:

Verwenden einer Mehrzahl von Detektoren, um das zeitlich veränderliche Magnetfeld eines Bereichs des Körpers eines Subjekts zu detektieren;
wobei einige oder alle der Detektoren in einer Gradiometerkonfiguration angeordnet sind.

6. Verfahren nach einem der vorstehenden Ansprüche, wobei jeder Detektor eine planare Spule umfasst.

7. Verfahren nach einem der vorstehenden Ansprüche, wobei das Magnetometersystem so konfiguriert ist, dass Detektorrauschen und Systemrauschen in jedem Signal, das an den Digitalisierer zur Digitalisierung bereitgestellt wird, weniger als etwa das Zweifache des Intervalls zwischen Digitalisierungsniveaus des Digitalisierers beträgt.

8. Verfahren nach einem der vorstehenden Ansprüche, wobei das Magnetometersystem so konfiguriert ist, dass das Detektorrauschen und Systemrauschen in jedem Signal weißes Rauschen umfasst oder sich diesem annähert.

9. Verfahren nach einem der vorstehenden Ansprüche, wobei jeder Detektor eine Spule oder eine Mehrzahl von in Reihe geschalteten Spulen umfasst und eine Konfiguration aufweist, sodass der Widerstand der Spule oder der Mehrzahl von in Reihe geschalteten Spulen im Bereich von 5 bis 3000 Ohm liegt.

10. Verfahren nach einem der vorstehenden Ansprüche, weiter umfassend das Verwenden eines Rauschgenerators, um zumindest einen Teil des Rauschens in jedem Signal zu erzeugen.

11. Verfahren nach einem der vorstehenden Ansprüche, wobei der Bereich des Körpers des Subjekts, dessen Magnetfeld analysiert wird, einen umfasst von: der Blase, dem Herzen, dem Kopf, dem Gehirn, der Gebärmutter oder einen Fötus.

12. Verfahren zum Analysieren des Magnetfeldes des Herzens eines Subjekts, wobei das Verfahren umfasst:
Verwenden des Verfahrens nach einem der vorstehenden Ansprüche, um das zeitlich veränderliche Magnetfeld des Herzens eines Subjekts zu analysieren.

13. Magnetometersystem (30) zur medizinischen Verwendung, umfassend:

einen oder mehreren Detektoren (60) zum Detektieren des zeitlich veränderlichen Magnetfelds eines Bereichs des Körpers eine Subjekts;
einen Digitalisierer (42), konfiguriert, um ein Signal oder Signale von dem einen oder den mehreren Detektoren zu digitalisieren, wobei jedes Signal, das digitalisiert wird, Rauschen und ein periodisches Signal einschließt, das von einem oder mehreren des einen oder der mehreren Detektoren aufgrund des zeitlich veränderlichen Magnetfelds des Bereichs des Körpers des Subjekts erzeugt wird; und
Mittelwertbildungsschaltung, konfiguriert, um das digitalisierte Signal oder die digitalisierten Signale über eine Mehrzahl von Perioden zu mitteln;
wobei das Magnetometersystem so konfiguriert ist, dass das Detektorrauschen und Systemrauschen in jedem Signal, das dem Digitalisierer zur Digitalisierung bereitgestellt wird, größer als etwa 25 % des Intervalls zwischen Digitalisierungsniveaus des Digitalisierers ist, sodass das Magnetometersystem ein oder mehrere Signalmerkmale des periodischen Signals detektieren kann, das von einem oder mehreren des einen oder der mehreren Detektoren aufgrund des zeitlich veränderlichen Magnetfelds des Bereichs des Körpers des Subjekts erzeugt wird, die kleiner als das Intervall zwischen Digitalisierungsniveaus des Digitalisierers sind.

14. Magnetometersystem nach Anspruch 13, wobei das Magnetometersystem so konfiguriert ist, dass das Detektorrauschen und Systemrauschen in jedem Signal, das an den Digitalisierer zur Digitalisierung bereitgestellt wird, weniger als etwa das Zweifache des Intervalls zwischen Digitalisierungsniveaus des Digitalisierers beträgt.

15. Magnetometersystem nach Anspruch 13 oder 14, wobei jeder Detektor eine Spule oder Mehrzahl von in Reihe geschalteten Spulen umfasst und eine Konfiguration aufweist, sodass der Widerstand der Spule oder der Mehrzahl von in Reihe geschalteten Spulen im Bereich von 5 bis 3000 Ohm liegt.

## Revendications

1. Procédé d'utilisation d'un système magnétomètre (30) pour analyser le champ magnétique d'une région d'un corps d'un sujet, le procédé comprenant :

l'utilisation d'un ou plusieurs détecteurs (60) pour détecter le champ magnétique qui varie dans le temps d'une région d'un corps d'un sujet ;
l'utilisation d'un numériseur (42) pour numériser un signal ou des signaux provenant du ou des détecteurs, chaque signal qui est numérisé incluant un bruit et un signal périodique produit par un ou plusieurs du ou des détecteurs en raison du champ magnétique qui varie dans le temps de la région du corps du sujet ; et
la pondération du signal ou des signaux numérisés sur plusieurs périodes ;
dans lequel le système magnétomètre est configuré de telle manière que le bruit du détecteur et le bruit du système dans chaque signal délivré au numériseur en vue de la numérisation est supérieur à environ 25 % de l'intervalle entre les niveaux de numérisation du numériseur, de telle manière que le système magnétomètre peut détecter une ou plusieurs caractéristiques de signal du signal périodique produit par un ou plusieurs du ou des détecteurs en raison du champ magnétique qui varie dans le temps de la région du corps du sujet qui sont inférieures à l'intervalle entre les niveaux de numérisation du numériseur.

2. Procédé selon la revendication 1, comprenant : l'utilisation d'un ou plusieurs réseaux de plusieurs détecteurs pour détecter le champ magnétique qui varie dans le temps d'une région d'un corps d'un sujet.

3. Procédé selon la revendication 1 ou 2, comprenant : l'utilisation de plusieurs couches de détecteurs pour détecter le champ magnétique qui varie dans le temps d'une région d'un corps d'un sujet.

4. Procédé selon la revendication 1, 2 ou 3, comprenant :

l'utilisation de plusieurs détecteurs pour détecter le champ magnétique qui varie dans le temps d'une région d'un corps d'un sujet ;
dans lequel certains ou tous les détecteurs sont connectés en parallèle et/ou en série.

5. Procédé selon l'une quelconque des revendications précédentes, comprenant :

l'utilisation de plusieurs détecteurs pour détecter le champ magnétique qui varie dans le temps d'une région d'un corps d'un sujet ;
dans lequel certains ou tous les détecteurs sont agencés dans une configuration de gradiomètre.

6. Procédé selon l'une quelconque des revendications précédentes, dans lequel chaque détecteur comprend une bobine plane.

7. Procédé selon l'une quelconque des revendications précédentes, dans lequel le système magnétomètre est configuré de telle manière que le bruit du détecteur et le bruit du système dans chaque signal délivré au numériseur en vue de la numérisation est inférieur à environ deux fois l'intervalle entre les niveaux de numérisation du numériseur.

8. Procédé selon l'une quelconque des revendications précédentes, dans lequel le système magnétomètre est configuré de telle manière que le bruit du détecteur et le bruit du système dans chaque signal comprend ou se rapproche du bruit blanc.

9. Procédé selon l'une quelconque des revendications précédentes, dans lequel chaque détecteur comprend une bobine ou plusieurs bobines connectées en série et a une configuration telle que la résistance de la bobine ou des plusieurs bobines connectées en série est dans la plage de 5 à 3 000 Ohms.

10. Procédé selon l'une quelconque des revendications précédentes, comprenant en outre l'utilisation d'un générateur de bruit pour générer au moins une partie du bruit dans chaque signal.

11. Procédé selon l'une quelconque des revendications précédentes, dans lequel la région du corps du sujet dont le champ magnétique est analysé comprend l'un parmi : la vessie, le cœur, la tête, le cerveau, l'utérus ou un fœtus.

12. Procédé d'analyse du champ magnétique d'un cœur d'un sujet, le procédé comprenant :
l'utilisation du procédé selon l'une des revendications précédentes pour analyser le champ magnétique qui varie dans le temps d'un cœur d'un sujet.

13. Système magnétomètre (30) à usage médical, comprenant :

un ou plusieurs détecteurs (60) pour détecter le champ magnétique qui varie dans le temps d'une région d'un corps d'un sujet ;
un numériseur (42) configuré pour numériser un signal ou des signaux provenant du ou des détecteurs, chaque signal qui est numérisé incluant un bruit et un signal périodique produit par un ou plusieurs du ou des détecteurs en raison du champ magnétique qui varie dans le temps de la région du corps du sujet ; et
la pondération de la circuiterie configurée pour pondérer le signal ou les signaux numérisés sur plusieurs périodes ;
dans lequel le système magnétomètre est configuré de telle manière que le bruit du détecteur et le bruit du système dans chaque signal délivré au numériseur en vue de la numérisation est supérieur à environ 25 % de l'intervalle entre les niveaux de numérisation du numériseur, de telle manière que le système magnétomètre peut détecter une ou plusieurs caractéristiques de signal du signal périodique produit par un ou plusieurs du ou des détecteurs en raison du champ magnétique qui varie dans le temps de la région du corps du sujet qui sont inférieures à l'intervalle entre les niveaux de numérisation du numériseur.

14. Système magnétomètre selon la revendication 13, dans lequel le système magnétomètre est configuré de telle manière que le bruit du détecteur et le bruit du système dans chaque signal délivré au numériseur en vue de la numérisation est inférieur à environ deux fois l'intervalle entre les niveaux de numérisation du numériseur.

15. Système magnétomètre selon la revendication 13 ou 14, dans lequel chaque détecteur comprend une bobine ou plusieurs bobines connectées en série et a une configuration telle que la résistance de la bobine ou des plusieurs bobines connectées en série est dans la plage de 5 à 3 000 Ohms.

FIG. 1

FIG. 2

FIG. 3

FIG. 4

FIG. 5

EP 3 448 250 B1

FIG. 6

FIG. 7

FIG. 8

FIG. 9

EP 3 448 250 B1

FIG. 10

FIG. 11

31

FIG. 12

EP 3 448 250 B1

FIG. 13

FIG. 14

FIG. 15

Single layer of spiral conductors
operating as a planar sensor

81

82

## FIG. 16

Multi-layer arrangement of spiral
conductors

81                          82

## FIG. 17

Example of ten layers connected in series

Example of ten layers connected 5 in series x 2 in parallel

Example of ten layers connected in series x 2 in parallel

## FIG. 18

Element 1

Element 2

## FIG. 19

FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015150475 A1
- US 2004260169 A1
- WO 2014011940 A2
- WO 2014006387 A